# FASCICULE DE BREVET EUROPEEN

(11) **EP 1 851 198 B1**
(45) Date de publication et mention de la délivrance du brevet: **31.12.2008**
(21) Numéro de dépôt: 06709274.2
(22) Date de dépôt: 08.02.2006
(51) Int. Cl.: C07D 207/34, A61K 31/40, A61P 35/00

(54) **PYRROLES SUBSTITUES COMPOSITIONS LES CONTENANT, PROCEDE DE FABRICATION ET UTILISATION**
SUBSTITUIERTE PYRROLE, ZUSAMMENSETZUNGEN, DIE DIESE ENTHALTEN, VERFAHREN ZUR IHRER HERSTELLUNG UND IHRE VERWENDUNG
SUBSTITUTED PYRROLES, COMPOSITIONS CONTAINING SAME, METHOD FOR MAKING SAME AND USE THEREOF

(30) Priorité: 10.02.2005 FR 0501354
(43) Date de publication de la demande: 07.11.2007
(73) Titulaire: Aventis Pharma S.A., 92160 Antony (FR)
(72) Inventeur: RONAN, Baptiste, F-92140 Clamart (FR); TABART, Michel, F-91290 La Norville (FR); SOUAILLE, Catherine, F-94600 Choisy le Roi (FR); VIVIANI, Fabrice, F-95380 Louvres (FR); BACQUE, Eric, F-91190 Gif sur Yvette (FR); LETTALEC, Jean-Philippe, F-75013 Paris (FR); DESMAZEAU, Pascal, F-91250 TIGERY (FR)
(74) Mandataire: Le Pennec, Magali
(86) Numéro de dépôt international: PCT/FR2006/000286
(87) Numéro de publication internationale: WO 2006/084996

(56) Documents cités:
- WO-A-02/079193
- WO-A-20/05049603
- US-A1- 2004 138 269
- US-A1- 2005 261 354

## Description

La présente invention concerne notamment de nouveaux composés chimiques, particulièrement de nouveaux pyroles substitués, les compositions les contenant, et leur utilisation comme médicaments.

Plus particulièrement, l'invention concerne de nouveaux pyrroles spécifiques présentant une activité anticancéreuse, via la modulation de l'activité de protéines, en particulier des kinases.

Il est connu selon la demande de brevet publiée sous le numéro WO02/079193 l'utilisation de pyrroles à visée anticancéreuses. Les dits composés sont des pyrroles substitués en position 2 par un groupe choisi parmi NH2, NO2, halogéne, CONH2, H ou CN et en position 4 par une pyrimidine susbtituée elle même par un groupe aminophényl. Aucune des dites pyrimidines n'est substituée par un groupe urée.

A ce jour, la plupart des composés commerciaux utilisés en chimiothérapie posent des problèmes importants d'effets secondaires et de tolérance par les patients. Ces effets pourraient être limités dans la mesure où les médicaments utilisés agissent sélectivement sur les cellules cancéreuses, à l'exclusion des cellules saines. Une des solutions pour limiter les effets indésirables d'une chimiothérapie peut donc consister en l'utilisation de médicaments agissant sur des voies métaboliques ou des éléments constitutifs de ces voies, exprimés majoritairement dans les cellules cancéreuses, et qui ne seraient pas ou peu exprimés dans les cellules saines.

Les protéines kinases sont une famille d'enzymes qui catalysent la phosphorylation de groupes hydroxyles de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire, la migration cellulaire ou la survie cellulaire. Parmi les différentes fonctions cellulaires dans lesquelles l'activité d'une protéine kinase est impliquée, certains processus représentent des cibles attractives pour traiter les maladies cancéreuses ainsi que d'autres maladies.

Ainsi, un des objets de la présente invention est de proposer des compositions ayant une activité anticancéreuse, en agissant en particulier vis-à-vis de kinases. Parmi les kinases pour lesquelles une modulation de l'activité est recherchée, FAK, KDR et Tie2 sont préférées.

Ces produits répondent à la formule (I) suivante : 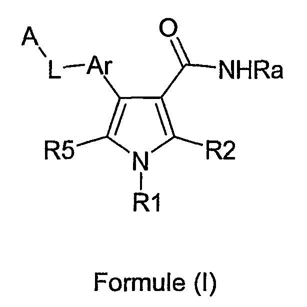 dans laquelle :
1) A et Ar sont indépendamment sélectionnés dans le groupe constitué par : aryle, hétéroaryle, hétérocyclyle, aryle substitué, hétéroaryle substitué, hétérocyclyle substitué, cycloalkyle, cycloalkyle substitué ;
2) L est sélectionné dans le groupe constitué par : NH, CO-NH, NH-CO, NH-SO₂, SO₂NH, NH-CH₂, CH₂-NH, CH₂-CO-NH, NH-CO-CH₂, NH-CH₂-CO, CO-CH₂-NH, NH-CO-NH, NH-CS-NH, NH-CO-O, O-CO-NH, CH₂-NH-CO-NH, NH-CO-NH-CH₂, NH-CO-CH₂-CO-NH;
3) Ra est sélectionné dans le groupe constitué par H, alkyle et cycloalkyle.
4) R1 est sélectionné dans le groupe constitué par : H, R, COR, SO₂R, dans lequel R est choisi parmi H, OR"₄, NR"₅R"₆, (C1-C6)alkyle, cycloalkyle, hétérocyclyle, hétérocyclyle substitué, aryle, aryle substitué, hétéroaryle, hétéroaryle substitué, dans lequel R"4 est choisi parmi H, phényle, alkyle, et dans lequel R"5 et R"6 sont indépendamment sélectionnés dans le groupe constitué par H, R OR"₄, (C1-C6)alkyle, cycloalkyle, hétérocyclyle, hétérocyclyle substitué, aryle, aryle substitué, hétéroaryle, hétéroaryle substitué ou bien R"5 et R"6 sont liés entre eux pour former un cycle saturé de 5 à 8 chaînons contenant de 0 à 3 hétéroatomes choisis parmi O,S et N;
5) R2 et R5 sont indépendamment sélectionnés dans le groupe constitué par: H, halogène, R'2, CN, O(R'2), OC(O)(R'2), OC(O)N(R'2)(R'3), OS(O₂)(R'2), N(R'2)(R'3), N=C(R'2)(R'3), N(R'2)C(O)(R'3), N(R'2)C(O)O(R'3), N(R'4)C(O)N(R'2)(R'3), N(R'4)C(S)N(R'2)(R'3), N(R'2)S(O₂)(R'3), C(O)(R'2), C(O)O(R'2), C(O)N(R'2)(R'3), C(=N(R'3))(R'2), C(=N(OR'3))(R'2), S(R'2), S(O)(R'2), S(O₂)(R'2), S(O₂)O(R'2), S(O₂)N(R'2)(R'3) ; dans lequel chaque R'2, R'3, R'4 est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué; dans lequel, lorsque R'2 et R'3 sont chacun différents de H et simultanément présents sur R2 ou sur R3, ils peuvent être liés entre eux pour former un cycle contenant de 0 à 3 hétéroatomes choisis parmi O, S et N.

Des produits de formule (I) préférés répondent à la définition suivante : 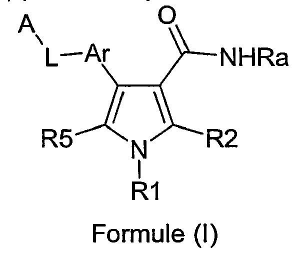 dans laquelle :
1) A et Ar sont sont tels que définis précédemment;
2) R1 est H :
3) L est sélectionné dans le groupe constitué par : NHCO, NH-CO-NH, NH, NHSO₂, NHCO-CH₂-CONH ;
4) Ra est sélectionné parmi H et méthyle.
5) R2 et R5 sont tels que définis précédemment.

Dans les produits de formule (I), Ar- L-A est avantageusement : 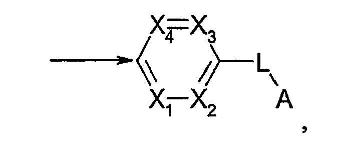 dans lequel chaque X1, X2, X3 et X4 est indépendamment choisi parmi N et C-R'5, dans lequel R'5 a la même définition que R2.

Des substituants R'5 sélectionnés dans le groupe constitué par H, F, Cl, méthyle, NH₂, OMe, OCF₃, et CONH₂ sont préférés.

Des substituants R2 et R5 préférés sont indépendamment sélectionnés dans le groupe constitué par: H, halogène, R'2, OR'2, NHR'2, NHCOR'2, NHCONHR'2, NHSO₂R'2. R2 et R5 sont préférentiellement H.

Un substituant Ra préféré est H.

Des substituants L-A préférés sont avantageusement choisis parmi NH-CO-NH-A et NH-SO₂-A.

Une combinaison L-A particulièrement efficace est obtenue lorsque L-A est NHCONH-A.

Des produits conformes à l'invention ont de préférence un substituant A qui est sélectionné dans le groupe constitué par phényle, pyridyle, pyrimidyle, thiényle, furyle, pyrrolyle, oxazolyle, thiazolyle, isoxazolyle, isothiazolyle, pyrazolyle, imidazolyle, indolyle, indazolyle, benzimidazolyle, benzoxazolyle, et benzothiazolyle ; éventuellement substitué.

De manière plus préférée, A est choisi parmi phényle, pyrazolyle et isoxazolyle ; éventuellement substitué.

Le substituant A est très avantageusement substitué par un premier substituant sélectionné dans le groupe constitué par alkyle, alkylène, alkynyle, aryle, hétéroaryle, O-alkyle, O-Aryle, O-hétéroaryle, S-alkyle, S-Aryle, S-hétéroaryle, chacun étant éventuellement substitué par un substituant choisi parmi (C1-C3)alkyle, halogène, O-(C1-C3)alkyle.

Le substituant A est préférentiellement substitué par un deuxième substituant sélectionné dans le groupe constitué par F, Cl, Br, I, OH, SH, SO₃M, COOM, CN, NO₂, CON(R8)(R9), N(R8)CO(R9), (C1-C3)alkyle-OH, (C1-C3)alkyleN(R8)(R9), (C1-C3)alkyle-(R10), (C1-C3)alkyle-COOH, N(R8)(R9); dans lequel R8 et R9 sont indépendamment choisis parmi H, (C1-C3)alkyle, (C1-C3)alkyle halogéné, (C1-C3)alkyleOH, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons contenant de 0 à 3 hétéroatomes ; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

Des substituants A particulièrement préférés sont choisis parmi phényle, pyrazolyle et isoxazolyle ; lesdits substituants A pouvant être substitués par halogène, (C1-C4)alkyle, (C1-C3)alkyle halogéné, O-(C1-C4)alkyle, S-(C1-C4)alkyle, O-(C1-C4)alkyle halogéné, et S-(C1-C4)alkyle halogéné. Lorsque A est disubstitué, les deux substituants de A peuvent former un cycle de 5 à 7 chaînons contenant de 0 à 3 hétéroatomes.
Les produits des exemples 1 à 41 sont objet de la présente invention.

Un produit conforme à l'invention pourra se présenter sous forme :
1) non chirale, ou
2) racémique, ou
3) enrichie en un stéréoisomère, ou
4) enrichie en un énantiomère ;
et pourra être éventuellement salifié.

Un produit conforme à l'invention pourra être utilisé pour la fabrication d'un médicament utile pour traiter un état pathologique, en particulier un cancer.

La présente invention concerne aussi les compositions thérapeutiques comprenant un produit selon l'invention, en combinaison avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront, de préférence, injectables et de ce fait auront une formulation acceptable pour une telle utilisation.

Des voies d'administration par injection acceptables incluent les voies intraveineuse, intra-péritonéale, intramusculaire, et sous cutanée, la voie intraveineuse étant habituellement préférée.

La dose administrée des composés de l'invention sera adaptée par le praticien en fonction de la voie d'administration au patient et de l'état de ce dernier.

Les composés de la présente invention peuvent être administrés seuls ou en mélange avec d'autres anticancéreux. Parmi les associations possibles on peut citer:
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
- les dérivés du platine comme notamment le cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoïdes (paclitaxel et docétaxel)
- les anthracyclines comme notamment la doxorubicine, la daunorubicine, l'idarubicine, l'épirubicine, la mitoxantrone, la losoxantrone
- les inhibiteurs de topoisomérases des groupes I et II telles que l'étoposide, le teniposide, l'amsacrine, l'irinotecan, le topotecan et le tomudex
- les fluoropyrimidines telles que la 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine tels que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la dexrazoxane, l'amifostine, l'herceptine ainsi que les hormones oestrogéniques, androgéniques
- les agents antivasculaires tels que les dérivés de la combretastatine, par exemple la CA4P, des chalcones ou de la colchicine, par exemple le ZD6126, et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

Les produits de l'invention sont utiles comme agents inhibiteurs d'une réaction catalysée par une kinase. FAK , KDR et Tie2 sont des kinases pour lesquelles les produits de l'invention seront particulièrement utiles en tant qu'inhibiteurs.

Les raisons pour lesquelles ces kinases sont choisies sont données ci-après :
FAK
   FAK est une tyrosine kinase cytoplasmique jouant un rôle important dans la transduction du signal transmis par les intégrines, famille de récepteurs hétérodimériques de l'adhésion cellulaire. FAK et les intégrines sont colocalisés dans des structures périmembranaires appelées plaques d'adhérence. Il a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33): 23439-442. (1992)]. L'autophosphorylation sur la tyrosine 397 de FAK représente un site de liaison pour une autre tyrosine kinase, Src, via son domaine SH2 [Schaller et al. Mol. Cell. Biol. 14 :1680-1688. 1994; Xing et al. Mol. Cell. Biol. 5 :413-421. 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature; 372:786-791. 1994; Schlaepfer et al. Prog. Biophy. Mol. Biol. 71:435-478. 1999; Schlaepfer and Hunter, J. Biol. Chem. 272:13189-13195. 1997]. L'activation de FAK peut aussi induire la voie de signalisation jun NH2-terminal kinase (JNK) et résulter dans la progression des cellules vers la phase G1 du cycle cellulaire [Oktay et al., J. Cell. Biol.145 :1461-1469. 1999]. Phosphatidylinositol-3-OH kinase (PI3-kinase) se lie aussi à FAK sur la tyrosine 397 et cette interaction pourrait être nécessaire à l'activation de PI3-kinase [Chen and Guan, Proc. Nat. Acad. Sci. USA. 91: 10148-10152. 1994; Ling et al. J. Cell. Biochem. 73 :533-544. 1999]. Le complexe FAK/Src phosphoryle différents substrats comme la paxilline et p130CAS dans les fibroblastes [Vuori et al. Mol. Cell. Biol. 16: 2606-2613. 1996].
   Les résultats de nombreuses études soutiennent l'hypothèse que les inhibiteurs de FAK pourraient être utiles dans le traitement du cancer. Des études ont suggéré que FAK pourrait jouer un rôle important dans la prolifération et/ou la survie cellulaires *in vitro.* Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143:1997-2008. 1998]. D'autres auteurs ont montré que des cellules tumorales traitées avec des oligonucleotides anti-sens de FAK perdent leur adhésion et entrent en apoptose (Xu et al, Cell Growth Differ. 4:413-418. 1996). Il a également été démontré que FAK promeut la migration des cellules *in vitro.* Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112:2677-91. 1999]. La surexpression du domaine C-terminal de FAK (FRNK) bloque l'étirement des cellules adhérentes et réduit la migration cellulaire *in vitro* [Richardson A. and Parsons J.T. Nature. 380:538-540. 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires *in vitro,* suggère le rôle potentiel de FAK dans les processus néoplasiques. Une étude récente a effectivement démontré l'augmentation de la prolifération des cellules tumorales *in vivo* après induction de l'expression de FAK dans des cellules d'astrocytome humain [Cary L.A. et al. J. Cell Sci. 109:1787-94. 1996; Wang D et al. J. Cell Sci. 113:4221-4230. 2000]. De plus, des études immunohistochimiques de biopsies humaines ont démontré que FAK était surexprimé dans les cancers de la prostate, du sein, de la thyroïde, du colon, du mélanome, du cerveau et du poumon, le niveau d'expression de FAK étant directement corrélé aux tumeurs présentant le phénotype le plus agressif [Weiner TM, et al. Lancet. 342(8878):1024-1025. 1993 ; Owens et al. Cancer Research. 55:2752-2755. 1995; Maung K. et al. Oncogene. 18:6824-6828. 1999; Wang D et al. J. Cell Sci. 113:4221-4230. 2000].
KDR
   KDR (Kinase insert Domain Receptor) aussi appelée VEGF-R2 (Vascular Endothelial Growth Factor Receptor 2), est exprimé uniquement dans les cellules endothéliales. Ce récepteur se fixe au facteur de croissance angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor : facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. Par conséquent, un inhibiteur sélectif de l'activité kinase du VEGF-R2 ne devrait démontrer que peu de toxicité.
   En plus de ce rôle central dans le processus dynamique angiogénique, des résultats récents suggèrent que l'expression de VEGF contribue à la survie des cellules tumorales après des chimio- et radio-thérapies, soulignant la synergie potentielle d'inhibiteurs de KDR avec d'autres agents (Lee et al. Cancer Research, 2000, vol. 60, p.5565-5570).
**Tie2**
   Tie-2 (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïetine 1 ou Ang1) qui stimule l'autophosphorylation du récepteur et la signalisation cellulaire [S. Davis et al (1996) Cell 87, 1161-1169] et l'antagoniste (angiopoïetine 2 ou Ang2) [P.C. Maisonpierre et al. (1997) Science 277, 55-60]. L'angiopoïetine 1 peut synergiser avec le VEGF dans les derniers stades de la néo-angiogénèse [AsaharaT. Circ. Res.(1998) 233-240]. Les expériences de knock-out et les manipulations transgéniques de l'expression de Tie2 ou de Ang1 conduisent à des animaux qui présentent des défauts de vascularisation [D.J. Dumont et al (1994) Genes Dev. 8, 1897-1909 *et* C. Suri (1996) Cell 87, 1171-1180]. La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P.C. Maisonpierre et al (1997) Science 277, 55-60]. Lin et al (1997) J. Clin. Invest. 100, 8: 2072-2078 *et* Lin P. (1998) PNAS 95, 8829-8834*,* ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénographes de tumeur du sein et de mélanome.
   Les inhibiteurs de Tie2 peuvent être utilisés dans les situations où une néovascularisation se fait de façon inappropriée (c'est-à-dire dans la rétinopathie diabétique, l'inflammation chronique, le psoriasis, le sarcome de Kaposi, la néovascularisation chronique due à la dégénérescence maculaire, l'arthrite rhumatoïde, l'hémoangiome infantile et les cancers).

### Définitions

Le terme « halogène » fait référence à un élément choisi parmi F, Cl, Br, et I.

Le terme « alkyle » fait référence à un substituant hydrocarboné saturé, linéaire ou ramifié, ayant de 1 à 12 atomes de carbone. Les substituants méthyle, éthyle, propyle, 1-méthyléthyl, butyle, 1-méthylpropyl, 2-méthylpropyle, 1,1-diméthyléthyle, pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, hexyle, 1-méthylpentyle, 2-méthylpentyle, 1-éthylbutyle, 2-éthylbutyle, 3,3-diméthylbutyle, heptyle, 1-éthylpentyle, octyle, nonyle, décyle, undécyle, et dodécyle sont des exemples de substituant alkyle.

Le terme « alkylène » fait référence à un substituant hydrocarboné linéaire ou ramifié ayant une ou plusieurs insaturations, ayant de 2 à 12 atomes de carbone. Les substituants éthylènyle, 1-méthyléthylènyle, prop-1-ènyle, prop-2-ènyle, Z-1-méthylprop-1-ènyle, E-1-méthylprop-1-ènyle, Z-1,2-diméthyl-prop-1-ènyle, E-1,2-diméthylprop-1-ènyle, but-1,3-diényle, 1-méthylidènyl-prop-2-ènyle, Z-2-méthylbut-1,3-diényle, E-2-méthylbut-1,3-diényle, 2-méthyl-1-méthylidènylprop-2-ènyle, undéc-1-ènyle et undéc-10-ènyle sont des exemples de substituant alkylène.

Le terme « alkynyle » fait référence à un substituant hydrocarboné linéaire ou ramifié ayant au moins deux insaturations portées par une paire d'atomes de carbone vicinaux, ayant de 2 à 12 atomes de carbone. Les substituants éthynyle; prop-1-ynyle; prop-2-ynyle; et but-1-ynyle sont des exemples de substituant alkynyle.

Le terme « aryle » fait référence à un substituant aromatique mono- ou polycyclique ayant de 6 à 14 atomes de carbone. Les substituants phényle, napht-1-yle ; napht-2-yle ; anthracen-9-yl ; 1,2,3,4-tétrahydronapht-5-yle ; et 1,2,3,4-tétrahydronapht-6-yle sont des exemples de substituant aryle.

Le terme « hétéroaryle » fait référence à un substituant hétéroaromatique mono- ou polycyclique ayant de 1 à 13 atomes de carbone et de 1 à 4 hétéroatomes. Les substituants pyrrol-1-yle ; pyrrol2-yle ; pyrrol3-yle ; furyle ; thienyle ; imidazolyle ; oxazolyle ; thiazolyle ; isoxazolyle ; isothiazolyle ; 1,2,4-triazolyle ; oxadiazolyle ; thiadiazolyle ; tétrazolyle ; pyridyle ; pyrimidyle ; pyrazinyle ; 1,3,5-triazinyle ; indolyle ; benzo[b]furyle ; benzo[b]thiényle ; indazolyle ; benzimidazolyle ; azaindolyle ; quinoléyle ; isoquinoléyle ; carbazolyle ; et acridyle sont des exemples de substituant hétéroaryle.

Le terme « hétéroatome » fait référence ici à un atome au moins divalent, différent du carbone. N; O; S; et Se sont des exemples d'hétéroatome.

Le terme « cycloalkyle » fait référence à un substituant hydrocarboné cyclique saturé ou partiellement insaturé ayant de 3 à 12 atomes de carbone. Les substituants cyclopropyle; cyclobutyle; cyclopentyle; cyclopentènyle; cyclopentadiényle; cyclohexyle; cyclohexènyle; cycloheptyle; bicyclo[2.2.1]heptyle ; cyclooctyle; bicyclo[2.2.2]octyle ; adamantyle ; et perhydronapthyle sont des exemples de substituant cycloalkyle.

Le terme « hétérocyclyle » fait référence à un substituant hydrocarboné cyclique saturé ou partiellement insaturé ayant de 1 à 13 atomes de carbone et de 1 à 4 hétéroatomes. De préférence, le substituant hydrocarboné cyclique saturé ou partiellement insaturé sera monocyclique et comportera 4 ou 5 atomes de carbone et 1 à 3 hétéroatomes.

Le terme « substitué » fait référence à un ou plusieurs substituants différents de H, par exemple halogène; alkyle; aryle; hétéroaryle, cycloalkyle ; hétérocyclyle ; alkylène; alkynyle; OH ; O-alkyle; O-alkylène ; O-aryle; O-hétéroaryle; NH₂ ; NH-alkyle; NH-aryle; NH-hétéroaryle; N-alkyle-alkyle' ; SH ; S-alkyle; S-aryle; S(O₂)H ; S(O₂)-alkyle; S(O₂)-aryle; SO₃ SO₃-alkyle SO₃ aryle; CHO ; C(O)-alkyle; C(O)-aryle ; C(O)OH ; C(O)O-alkyle; C(O)O-aryle ; OC(O)-alkyle; OC(O)-aryle ; C(O)NH₂ ; C(O)NH-alkyle; C(O)NH-aryle ; NHCHO ; NHC(O)-alkyle; NHC(O)-aryle ; NH-cycloalkyle ; NH-hétérocyclyle.

La présente invention a encore pour objet le procédé de préparation des produits de formule (I).
Les produits selon l'invention peuvent être préparés à partir de méthodes conventionnelles de chimie organique.
Les schéma 1,2, 3 et 4 ci-dessous sont illustratifs des méthodes utilisées pour la préparation des exemples concernant les pyroles substitués. A ce titre, elles ne sauraient constituer une limitation de la portée de l'invention, en ce qui concerne les méthodes de préparation des composés revendiqués.

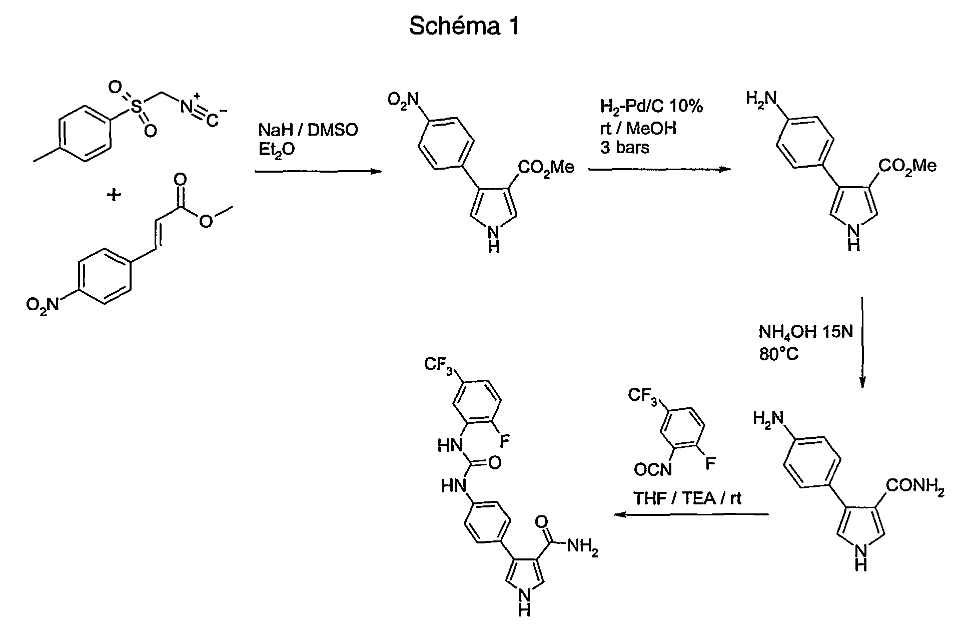

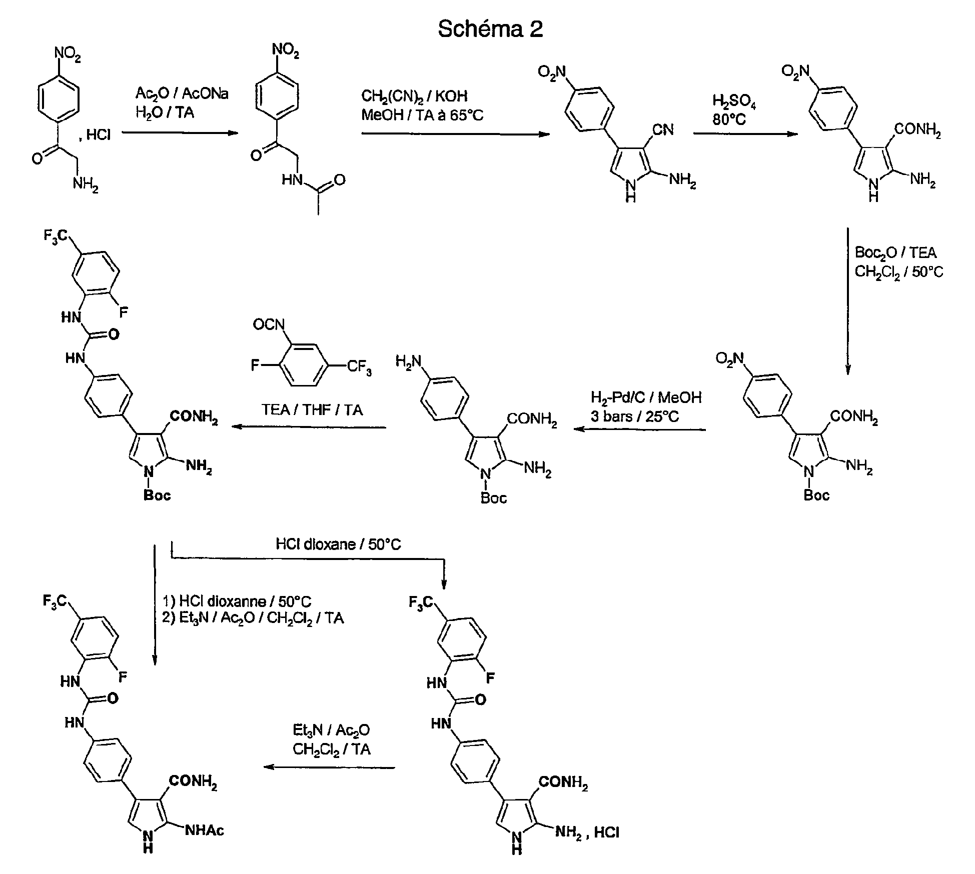

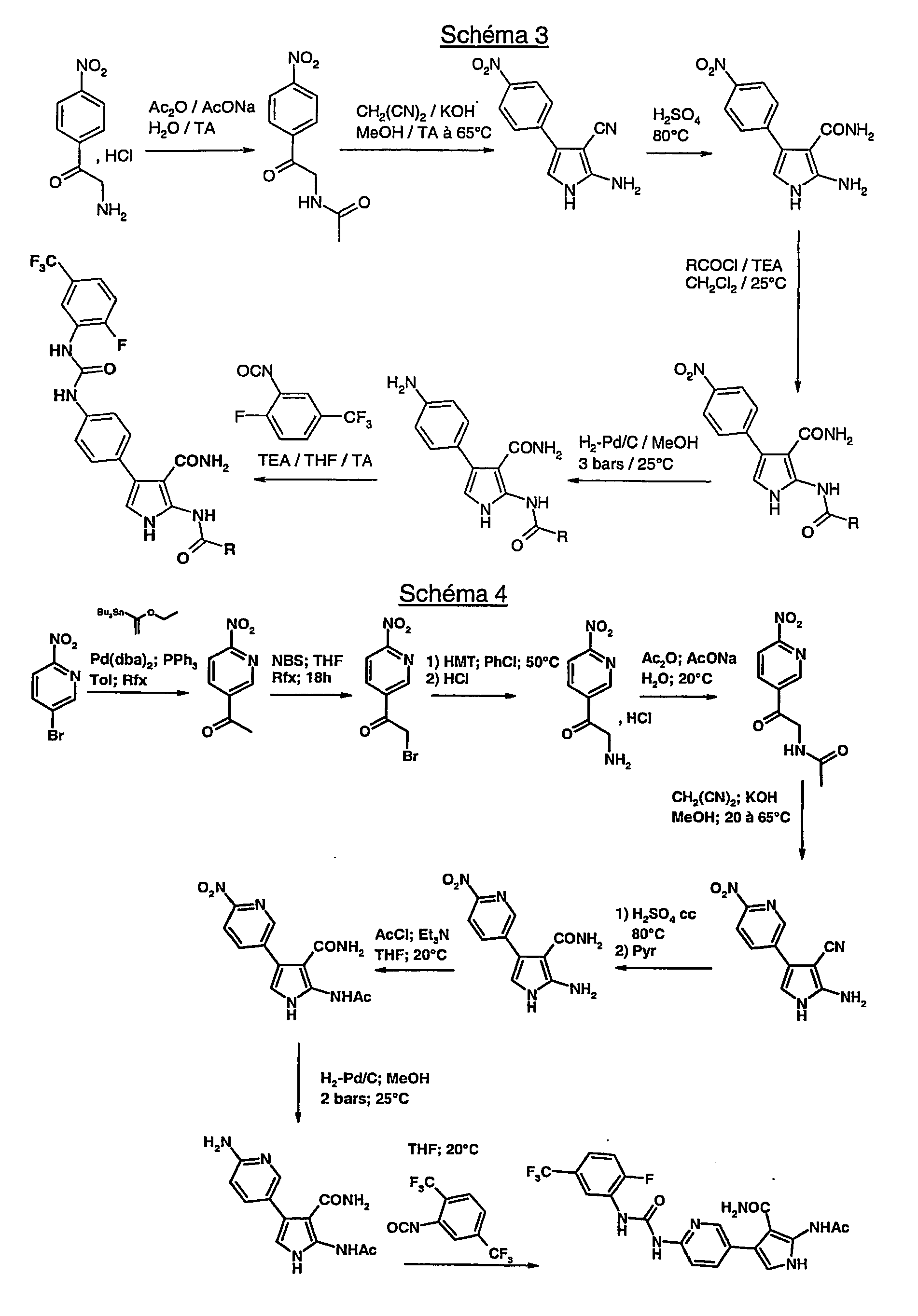

II est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires. Ces groupes sont ceux qui permettent d'être éliminés sans toucher au reste de la molécule. Comme exemples de groupes protecteurs de la fonction amino, on peut citer le carbamate de *tert*-butyle qui peut être régénéré au moyen d'acide trifluoroacétique ou d'iodotriméthylsilane, l'acétyle qui peut être régénéré en milieu acide (acide chlorhydrique par exemple). Comme groupes protecteurs de la fonction carboxyle, on peut citer les esters (méthoxyméthylester, benzylester par exemple). Comme groupes protecteurs de la fonction alcool, on peut citer les esters (benzoylester par exemple) qui peuvent être régénérés en milieu acide ou par hydrogénation catalytique. D'autres groupes protecteurs utilisables sont décrits par T. W. GREENE et coll. dans Protective Groups in Organic Synthesis, third edition, 1999, Wiley-Interscience.
Les composés de formule (I) sont isolés et peuvent être purifiés par les méthodes connues habituelles, par exemple par cristallisation, chromatographie ou extraction.
Les énantiomères, diastéréoisomères des composés de formule (I) font également partie de l'invention.
Les composés de formule (I) comportant un reste basique peuvent être éventuellement transformés en sels d'addition avec un acide minéral ou organique, par action d'un tel acide au sein d'un solvant, par exemple organique tel un alcool, une cétone, un éther ou un solvant chloré.
Les composés de formule (I) comportant un reste acide peuvent être éventuellement transformés en sels métalliques ou en sels d'addition avec des bases azotées selon les méthodes connues en soi. Ces sels peuvent être obtenus par action d'une base métallique (alcaline ou alcalinoterreuse par exemple), de l'ammoniac, d'une amine ou d'un sel d'amine sur un composé de formule (I), dans un solvant. Le sel formé est séparé par les méthodes habituelles.
Ces sels font également partie de l'invention.
Lorsqu'un produit selon l'invention présente au moins une fonction basique libre, des sels pharmaceutiquement acceptables peuvent être préparés par réaction entre ledit produit et un acide minéral ou organique. Des sels pharmaceutiquement acceptables incluent les chlorures, nitrates, sulfates, hydrogénosulfates, pyrosulfates, bisulfates, sulfites, bisulfites, phosphates, monohydrogénophosphates, dihydrogénophosphates, métaphosphates, pyrophosphates, acétates, propionates, acrylates, 4-hydroxybutyrates, caprylates, caproates, décanoates, oxalates, malonates, succinates, glutarates, adipates, pimélates, maléates, fumarates, citrates, tartrates, lactates, phénylacétates, mandélates, sébacates, subérates, benzoates, phtalates, méthanesulfonates, p-toluène sulfonate, propanesulfonates, xylènesulfonates, salicylates, cinnamates, glutamates, aspartates, glucuronates, galacturonates.
Lorsqu'un produit selon l'invention présente au moins une fonction acide libre, des sels pharmaceutiquement acceptables peuvent être préparés par réaction entre ledit produit et une base minérale ou organique. Des bases pharmaceutiquement acceptables incluent des hydroxydes de cations de métaux alcalins ou alcalino-terreux tels que Li, Na, K, Mg, Ca, des composés aminés basiques tels qu'ammoniac, arginine, histidine, pipéridine, morpholine, pipérazine, triéthylamine.

L'invention est également décrite par les exemples suivants, donnés à titre d'illustration de l'invention.

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses Mass spectra a été réalisée sur une gamme de 180 à 800. Les données ont été analysées en utilisant le logiciel Micromass MassLynx. La séparation a été effectuée sur une colonne Hypersil BDS C18, 3 µm (50 x 4.6 mm), en éluant par un gradient linéaire de 5 à 90% d'acétonitrile contenant 0,05% (v/v) d'acide trifluoroacétique (TFA) dans l'eau contenant 0,05% (v/v) TFA en 3,5 mn à un débit de 1 mL/mn. Le temps total d'analyse, incluant la période de rééquilibration de la colonne, est de 7 mn.
Les spectres MS ont été réalisés en électrospray (ES⁺) sur un appareil Platform II (Micromass). Les principaux ions observés sont décrits.
Les points de fusion ont été mesurés en capillaire, sur un appareil Mettler FP62, gamme 30°C à 300°C, montée de 2°C par minute.

### Purification par LC/MS:

Les produits peuvent être purifiés par LC/MS en utilisant un système Waters FractionsLynx composé d'une pompe à gradient Waters modèle 600, d'une pompe de régénération Waters modèle 515, d'une pompe de dilution Waters Reagent Manager, d'un auto-injecteur Waters modèle 2700, de deux vannes Rheodyne modèle LabPro, d'un détecteur à barrette de diodes Waters modèle 996, d'un spectromètre de masse Waters modèle ZMD et d'un collecteur de fractions Gilson modèle 204. Le système était controlé par le logiciel Waters FractionLynx. La séparation a été effectuée alternativement sur deux colonnes Waters Symmetry (C₁₈, 5µM, 19x50 mm, référence catalogue 186000210), une colonne étant en cours de régénération par un mélange eau/acétonitrile 95/5 (v/v) contenant 0,07% (v/v) d'acide trifluoroacétique, pendant que l'autre colonne était en cours de séparation. L'élution des colonnes a été effectuée en utilisant un gradient linéaire de 5 à 95% d'acétonitrile contenant 0,07% (v/v) d'acide trifluoroacétique dans l'eau contenant 0,07% (v/v) d'acide trifluoroacétique, à un débit de 10 mL/mn. A la sortie de la colonne de séparation, un millième de l'effluent est séparé par un LC Packing Accurate, dilué à l'alcool méthylique à un débit de 0,5 mL/mn et envoyé vers les détecteurs, à raison de 75% vers le détecteur à barrette de diodes, et les 25% restants vers le spectromètre de masse. Le reste de l'effluent (999/1000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Na]⁺. Dans certains cas, dépendant des résultats de LC/MS analytique, quand un ion intense correspondant à [M+2H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺⁺ et/ou [M+Na+H]⁺⁺ sont détectés. Les produits ont été collectés en tube de verre tarés. Après collecte, les solvants ont été évaporés, dans un évaporateur centrifuge Savant AES 2000 ou Genevac HT8 et les masses de produits ont été déterminées par pesée des tubes après évaporation des solvants.

### Analyse El-Cl : introduction directe (DCI=dépôt échantillon sur filament)

Spectromètre de masse Finnigan SSQ7000 ; domaine de masse m/z=29-900 ; énergie des électrons 70eV ;température de la source 70 °C ;gaz réactant Cl ammoniac ; El=lonisation par impact d'électrons ; CI= Ionisation chimique.
Analyse electrospray : (electrospray positif : ES⁺ ; electrospay négatif : ES⁻)
Couplage LC-MS-DAD-ELSD :
Méthode A
   MS : Waters-Micromass Platform II; LC : Agilent HP 1100 ; colonne Hypersil GOLD Thermo C18; 3X50 mm, 3 µm; éluant: gradient Eau (avec Acide Formique 0,1% + acetonitrile sur 7 mn; débit = 0.8 ml/mn ; UV : DAD ( λ=200-400nm).
Méthode B
   MS : Waters-Micromass QTOF-2; LC : Agilent HP 1100 ; colonne Hypersil GOLD Thermo C18; 3X50 mm, 3 µm; éluant: gradient Eau (avec Acide Formique 0,1 % ) + acetonitrile sur 7 mn; débit = 0.9 ml/mn ; UV : DAD ( λ=200-400nm).
Méthode C
   MS : Waters-Micromass ZQ; LC : Agilent HP 1100 ; colonne XBRIDGE Waters C18; 3X50 mm, 2.5 µm; éluant: gradient Eau (avec Acide Formique 0,1% ) + acetonitrile sur 7 mn; débit =1.1 ml/mn ; UV : DAD (λ=254nm).
   Spectre RMN 1 H à 400 MHz sur spectromètre BRUKER AVANCE DRX-400 ou à 300 MHz sur spectromètre BRUKER AVANCE DPX-300 avec les déplacements chimiques (δ en ppm) - dans le solvant diméthylsulfoxide - d6 (DMSO-d6) référencé à 2,50 ppm à la température de 303K.

### Exemple 1

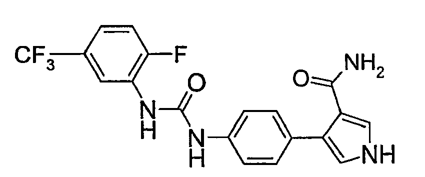

### 4-{4-[3-(2-Fluoro-5-trifluorométhyl-phényl)-uréido]-phényl}-1 H-pyrrole-3-carboxamide

A 0,017 g (84,48 mmol) de 4-(4-amino-phényl)-1H-pyrrole-3-carboxamide en suspension dans 27 cm³ de tétrahydrofurane, on ajoute à une température voisine de 20°C, sous atmosphère d'argon, 0,012 cm³ de 2-fluoro-5-trifluorométhyl-phényl isocyanate et 0,012 cm³ de triéthylamine. Après 20 heures d'agitation à une température voisine de 20°C, le mélange réactionnel est concentré à sec sous pression réduite (2,7 kPa) pour donner un résidu qui est purifié par chromatographie-flash [éluant : acétate d'éthyle /dichlorométhane (95 / 5 en volumes)]. Après concentration des fractions sous pression réduite, on obtient un résidu jaune qui est agité dans 5 cm³ de dichlorométhane puis filtré et séché sous pression réduite (2,7 kPa) pour donner 22 mg de 4-{4-[3-(2-fluoro-5-trifluoromethyl-phenyl)-ureido]-phenyl}-1 H-pyrrole-3-carboxamide, sous forme d'un solide beige; R.M.N. 1 H (300 MHz, (CD₃)₂SO, - δ en ppm) : de 6,57 à 7,02 (m très étalé : 2H) ; 6,85 (t large, J = 2,5 Hz : 1 H) ; 7,29 (t large, J = 2,5 Hz : 1H) ; de 7,33 à 7,43 (m : 5H) ; 7,49 (dd, J = 10,5 et 8,5 Hz : 1H); 8,60 (dd, J = 7,5 et 2,5 Hz : 1H); 9,31 (s large : 1 H) ; 9,58 (s large : 1 H) ; 11,2 (s large : 1 H) ; IE: m/z = 406 (M⁺), m/z=205 (C₈H₃NOF₄⁺), m/z=179 (C₇H₄NF₄⁺)pic de base, ES+: m/z = 407 (MH⁺).

Le 4-(4-amino-phényl)-1H-pyrrole-3-carboxamide peut être préparé de la manière suivante :
Une suspension de 0,07 g (0,304 mmol) de 4-(4-amino-phényl)-1H-pyrrole-3-carboxylate d'éthyle dans 10 cm³ d'une solution aqueuse d'ammoniaque à 22% est chauffée en autoclave à une température voisine de 80°C pendant 84 heures. Après arrêt du chauffage puis retour à température et pression ambiantes, le mélange réactionnel est concentré à sec sous pression réduite (2,7 kPa) pour donner un solide orange qui est purifié par chromatographie-flash [éluant : dichlorométhane / méthanol / acétonitrile (98 / 1 / 1 en volume)]. Après concentration des fractions sous pression réduite, on obtient un résidu qui est agité dans 10 cm³ d'éther diéthylique puis est filtré et séché sous pression réduite (2,7 kPa) pour donner 0,02 g de 4-(4-amino-phényl)-1 H-pyrrole-3-carboxamide, sous forme d'un solide marron; IE: m/z = 201 (M⁺)pic de base, m/z= 185 (M - NH2⁺), m/z= 157 (M - CONH2⁺).

Le 4-(4-amino-phényl)-1 H-pyrrole-3-carboxylate d'éthyle peut être préparé de la manière suivante :
A une suspension de 0,02 g (0,188 mmol) de palladium sur charbon à 10% dans 15 cm³ de méthanol, on ajoute à une température voisine de 20°C, 0,2 g (0,769 mmol) de 4-(4-nitro-phényl)-1 H-pyrrole-3-carboxylate d'éthyle. Après 20 heures d'hydrogénation en autoclave sous 3 bars d'hydrogène, à une température voisine de 25°C, le mélange réactionnel est filtré, le catalyseur est rincé avec 3 fois 5 cm³ de méthanol puis le filtrat est concentré à sec sous pression réduite (2,7 kPa) pour donner un résidu qui est agité dans 10 cm³ d'éther diéthylique puis est filtré et séché sous pression réduite (2,7 kPa) pour donner 0,079 g de 4-(4-amino-phényl)-1H-pyrrole-3-carboxylate d'éthyle, sous forme d'un solide marron; IE: m/z = 230 (M⁺)pic de base, m/z= 202 (M-C₂H₄⁺), m/z= 157 (M - C₂H₅O⁺).

Le 4-(4-nitro-phényl)-1H-pyrrole-3-carboxylate d'éthyle peut être préparé de la manière suivante :
A une suspension de 0,512 g (12,8 mmol) d'hydrure de sodium à 60% dans de l'huile minérale dans 20 cm³ d'éther diéthylique, on ajoute goutte à goutte, à une température voisine de 20°C, sous atmosphère d'argon, un mélange de 2,212 g (10 mmol) de 4-nitrocinnamate d'éthyle et 1,991 g (10,2 mmol) de tosyl-méthyl isocyanate en solution dans un mélange de 18 cm³ de diméthylsulfoxyde et 36 cm³ d'éther diéthylique. Après 1 heure d'agitation à reflux, le mélange réactionnel est repris dans un mélange de 70 cm³ d'eau, 20 cm³ d'une solution aqueuse saturée en chlorure de sodium et 100 cm³ d'acétate d'éthyle. La phase aqueuse est extraite par 50 cm³ d'éther diéthylique et 2 fois 75 cm³ de dichlorométhane. Toutes les phases organiques sont réunies, séchées sur sulfate de sodium anhydre, filtrées et concentrées à sec sous pression réduite (2,7 kPa) pour donner une huile noire qui est reprise dans un mélange de 75 cm³ d'eau et 50 cm³ d'acétate d'éthyle. La phase aqueuse est extraite par 2 fois 50 cm³ d'acétate d'éthyle. Toutes les phases organiques sont réunies, séchées sur sulfate de sodium anhydre, filtrées et concentrées à sec sous pression réduite (2,7 kPa) pour donner 2,82 g d'un solide noir qui est purifié par chromatographie-flash [éluant : cyclohexane / acétate d'éthyle (3 / 2 en volumes)]. Après concentration des fractions sous pression réduite, on obtient 1,48 g d'un solide orange qui est purifié par chromatographie-flash [éluant : dichlorométhane]. Après concentration des fractions sous pression réduite, on obtient 0,78 g de 4-(4-nitro-phényl)-1 H-pyrrole-3-carboxylate d'éthyle, sous forme d'un solide jaune ; IE: m/z = 260 (M⁺·) pic de base, m/z= 215 (M - C₂H₅O⁺), m/z=169 (215-NO₂).

### Exemple 2

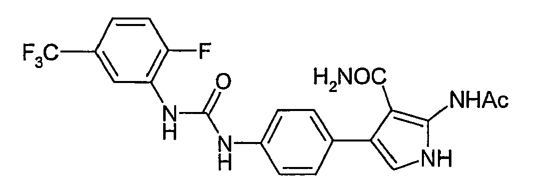

### 1-Acétyl-2-amino-4-{4-[3-(2-fluoro-5-trifluorométhyl-phényl)-ureido]-phényl}-1 H-pyrrole-3-carboxamide :

A 0,06 g (0,115 mmol) de 2-amino-3-carbamoyl-4-{4-[3-(2-fluoro-5-trifluorométhyl-phényl)-ureido]-phényl}-pyrrole-1-carboxylate de tert-butyle en solution dans un mélange de 1,2 cm³ de dioxane et 1,2 cm³ de méthanol, on ajoute à une température voisine de 20°C, sous atmosphère d'argon, 0,575 cm³ d'une solution d'acide chlorhydrique 4M dans le dioxane. Après 15 heures d'agitation à une température voisine de 50°C, le mélange réactionnel est concentré à sec sous pression réduite (2,7 kPa) pour donner le chlorhydrate du 2-amino-3-carbamoyl-4-{4-[3-(2-fluoro-5-trifluorométhyl-phényl)-ureido]-phényl}-1H-pyrrole qui est repris dans 2,5 cm³ d'acétate d'éthyle. On ajoute à une température voisine de 20°C, sous atmosphère d'argon, 0,01 cm³ de triéthylamine et 0,013 cm³ d'anhydride acétique. Après 1 heure d'agitation à une température voisine de 20°C, on ajoute une quantité catalytique de DMAP puis on maintient l'agitation pendant 30 minutes. Le mélange réactionnel est dilué par 5 cm³ d'acétate d'éthyle. La phase organique est lavée par 2 fois 5 cm³ d'eau. Toutes les phases aqueuses sont réunies et extraites par 5 cm³ d'acétate d'éthyle. Toutes les phases organiques sont réunies, lavées par 5 cm³ d'une solution aqueuse saturée en chlorure de sodium, séchées sur sulfate de magnésium anhydre, filtrées et concentrées à sec sous pression réduite (2,7 kPa) pour donner 0,054 g d'un résidu qui est purifié par chromatographie-flash [éluant : dichlorométhane /méthanol (98/ 2 en volumes)]. Après concentration des fractions sous pression réduite, on obtient 0,010 g de 1-acétyl-2-amino-4-{4-[3-(2-fluoro-5-trifluorométhyl-phényl)-ureido]-phényl}-1 H-pyrrole-3-carboxamide, sous forme d'un solide jaune; R.M.N. 1H (300 MHz, (CD₃)₂SO, - δ en ppm) : 2,14 (s : 3H) ; de 5,20 à 6,10 (m étalé : 1 H) ; 6,40 (d, J = 2,0 Hz : 1H) ; de 6,70 à 7,60 (m étalé : 1 H) ; 7,30 (d large, J = 8,5 Hz : 2H) ; 7,38 (mt : 1H) ; de 7,45 à 7,54 (m : 3H) ; 8,62 (dd, J = 7,5 et 2,5 Hz : 1 H) ; 8,92 (s large : 1H) ; 9,25 (s large : 1 H) ; 10,7 (s large : 1 H) ; 11,4 (s large : 1H) ; ES+: m/z = 464 (MH⁺).

Le 2-amino-3-carbamoyl-4-{4-[3-(2-fluoro-5-trifluorométhyl-phényl)-ureido]-phényl}-pyrrole-1-carboxylate de tert-butyle peut être préparé de la manière suivante :
A 0,07 g (0,221 mmol) de 2-amino-4-(4-amino-phényl)-3-carbamoyl-pyrrole-1-carboxylate de tert-butyle en solution dans 2 cm³ de tétrahydrofurane, on ajoute à une température voisine de 20°C, sous atmosphère d'argon, 0,125 cm³ de triéthylamine et 0,049 cm³ de 2-fluoro-5-trifluorométhyl-phényl isocyanate. Après 4 heures d'agitation à une température voisine de 20°C, le mélange réactionnel est repris dans 5 cm³ de dichlorométhane. La phase organique est lavée par 2 fois 5 cm³ d'eau. Toutes les phases aqueuses sont réunies et extraites par 5 cm³ de dichlorométhane. Toutes les phases organiques sont réunies, lavées par 5 cm³ d'une solution aqueuse saturée en chlorure de sodium, séchées sur sulfate de magnésium anhydre, filtrées et concentrées à sec sous pression réduite (2,7 kPa) pour donner 0,135 g d'un solide orange qui est purifié par chromatographie-flash [éluant : dichlorométhane / méthanol (100/ 0 à 98 /2 en volume)]. Après concentration des fractions sous pression réduite, on obtient 0,077 g de 2-amino-3-carbamoyl-4-{4-[3-(2-fluoro-5-trifluorométhyl-phényl)-ureido]-phényl}-pyrrole-1-carboxylate de tert-butyle, sous forme d'un solide jaune; ES+: m/z = 522 (MH⁺).

Le 2-amino-4-(4-amino-phényl)-3-carbamoyl-pyrrole-1-carboxylate de tert-butyle peut être préparé de la manière suivante :
A une suspension de 0,008 g (0,0076 mmol) de palladium sur charbon à 10% dans 12 cm³ de méthanol, on ajoute à une température voisine de 25°C, 0,075 g (0,216 mmol) de 2-amino-3-carbamoyl-4-(4-nitro-phényl)-pyrrole-1-carboxylate de tert-butyle. Après 17 heures d'hydrogénation en autoclave sous 3 bars d'hydrogène, à une température voisine de 25°C, le mélange réactionnel est filtré, le catalyseur est rincé avec 3 fois 5 cm³ de méthanol puis le filtrat est concentré à sec sous pression réduite (2,7 kPa) pour donner 0,072 g de 2-amino-4-(4-amino-phényl)-3-carbamoyl-pyrrole-1-carboxylate de tert-butyle, sous forme d'un solide jaune; R.M.N. 1 H (400 MHz, (CD₃)₂SO, - δ en ppm) : 1,55 (s : 9H) ; de 4,80 à 5,35(m très étalé : 1 H) ; 5,22 (s large : 2H) ; 6,32 (s : 1 H) ; de 6,40 à 6,85 (m très étalé : 1H) ; 6,60 (d large, J = 8,5 Hz : 2H) ; 6,99 (d large, J = 8,5 Hz : 2H) ; 7,01 (s large : 2H).

Le 2-amino-3-carbamoyl-4-(4-nitro-phényl)-pyrrole-1-carboxylate de tert-butyle peut être préparé de la manière suivante :
A 0,11 g (0,447 mmol) de 2-amino-4-(4-nitro-phényl)-1 H-pyrrole-3-carboxamide en suspension dans 6 cm³ de dichlorométhane, on ajoute à une température voisine de 20°C, sous atmosphère d'argon, 0,075 cm³ (0,536 mmol) de triéthylamine puis 0,117 g (0,536 mmol) de di-tertbutyl-dicarbonate. Après 2,5 heures d'agitation à une température voisine de 50°C, on ajoute 0,09 g (0,412 mmol) de di-tertbutyl-dicarbonate et on maintient l'agitation à une température voisine de 50°C pendant 3 heures. Le mélange réactionnel est repris dans 5 cm³ de dichlorométhane. La phase organique est lavée par 3 fois 5 cm³ d'eau. Toutes les phases aqueuses sont réunies et extraites par 5 cm³ de dichlorométhane. Toutes les phases organiques sont réunies, séchées sur sulfate de magnésium anhydre, filtrées et concentrées à sec sous pression réduite (2,7 kPa) pour donner 0,28 g d'un résidu qui est purifié par chromatographie-flash [éluant : dichlorométhane]. Après concentration des fractions sous pression réduite, on obtient 0,075 g de 2-amino-3-carbamoyl-4-(4-nitro-phényl)-pyrrole-1-carboxylate de tert-butyle, sous forme d'un solide orange; R.M.N. 1H (300 MHz, (CD₃)₂SO, - δ en ppm) : 1,57 (s : 9H) ; de 5,77 à 6,58 (m étalé : 2H) ; 6,72 (s : 1H) ; 6,93 (s large : 2H) ; 7,63 (d large, J = 8,5 Hz : 2H) ; 8,23 (d large, J = 8,5 Hz : 2H).

Le 2-amino-4-(4-nitro-phényl)-1 H-pyrrole-3-carboxamide peut être préparé de la manière suivante :
Une suspension de 0,26 g (1,139 mmol) de 2-amino-4-(4-nitro-phényl)-1H-pyrrole-3-carbonitrile dans 5 cm³ d'acide sulfurique concentré, est chauffée à une température voisine de 80°C pendant 1 heure. Après avoir refroidi le mélange réactionnel à une température voisine de 20°C, on verse celui-ci sur de la glace pilée puis on ajoute lentement une solution aqueuse de soude 5N jusqu'à un pH basique voisin de 10. Le mélange réactionnel est extrait par 7 fois 10 cm³ d'un mélange de dichlorométhane / MeOH (98 / 2 en volume). Toutes les phases organiques sont réunies, séchées sur sulfate de magnésium anhydre, filtrées et concentrées à sec sous pression réduite (2,7 kPa) pour donner 0,19 g de 2-amino-4-(4-nitro-phényl)-1H-pyrrole-3-carboxamide, sous forme d'un solide brun-rouge; IE: m/z = 246 (M⁺˙)pic de base, m/z= 229 (M - NH₃⁺).

Le 2-amino-4-(4-nitro-phényl)-1H-pyrrole-3-carbonitrile peut être préparé de la manière suivante :
A 0,5 g (2,25 mmol) de N-[2-(4-nitro-phényl)-2-oxo-ethyl]-acétamide en solution dans 15 cm³ de méthanol, on ajoute à une température voisine de 20°C, sous atmosphère d'argon, 0,223 g (3,375 mmol) de malononitrile. Le milieu réactionnel est refroidi à une température voisine de 0°C puis 0,5 cm³ d'une solution aqueuse à 50% d'hydroxyde de potassium est ajoutée. Après 15 minutes d'agitation à une température voisine de 0°C puis 30 minutes d'agitation à une température voisine de 65°C, le mélange réactionnel est refroidi à une température voisine de 20°C puis versé sur de la glace pilée et extrait par 7 fois 10 cm³ de dichlorométhane. Toutes les phases organiques sont réunies, lavées par 50 cm³ d'une solution aqueuse saturée en chlorure de sodium, séchées sur sulfate de magnésium anhydre, filtrées et concentrées à sec sous pression réduite (2,7 kPa) pour donner 0,7 g d'un solide brun qui est purifié par chromatographie-flash [éluant : dichlorométhane / méthanol (100/ 0 à 95 / 5 en volumes)]. Après concentration des fractions sous pression réduite, on obtient 0,265 g de 2-amino-4-(4-nitro-phényl)-1H-pyrrole-3-carbonitrile, sous forme d'un solide brun; IE: m/z = 228 (M⁺˙)pic de base, m/z=182 (M-NO₂).

Le N-[2-(4-nitro-phényl)-2-oxo-éthyl]-acétamide peut être préparé de la manière suivante :
A 0,5 g (2,308 mmol) de 2-amino-(4-nitrophényl)-acétophénone en suspension dans 2 cm³ d'eau, on ajoute à une température voisine de 0°C, sous atmosphère d'argon, 0,435 cm³ (4,616 mmol) d'anhydride acétique puis 0,379 g (4,616 mmol) d'acétate de sodium en solution dans 1,5 cm³ d'eau. Après avoir laissé évoluer la température entre 0°C et 20°C pendant 1 heure, on ajoute 1,5 cm³ d'acide chlorhydrique concentré jusqu'à un pH = 2. le mélange réactionnel est extrait par 5 fois 10 cm³ de dichlorométhane. Toutes les phases organiques sont réunies, séchées sur sulfate de magnésium anhydre, filtrées et concentrées à sec sous pression réduite (2,7 kPa) pour donner 0,37 g de N-[2-(4-nitro-phényl)-2-oxo-ethyl]-acétamide, sous forme d'un solide jaune; ES+: m/z = 223 (MH⁺).

### Exemple 3

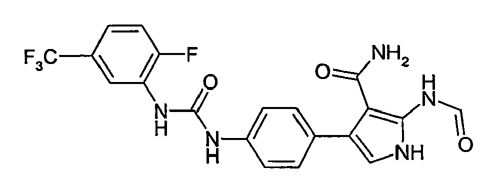

2-Formylamino-4-{4-[3-(2-fluoro-5-trifluorométhyl-phényl)-uréido]-phényl}-1H-pyrrole-3-carboxamide :
   A 0,125 g (0,578 mmol) de 2-formylamino-4-(4-amino-phényl)-1 H-pyrrole-3-carboxamide en solution dans 15 cm³ de tétrahydrofurane, on ajoute à une température voisine de 23°C, 0,081 cm³ de triéthylamine et 0,084 cm³ de 2-fluoro-5-trifluorométhyl-phényl isocyanate. Après 16 heures d'agitation à une température voisine de 23°C, le mélange réactionnel est concentré à sec sous pression réduite (2,7 kPa). Le résidu est alors dilué dans 50cm³ d'acétate d'éthyle puis lavé par 2 fois 50 cm³ d'eau et 50 cm³ d'une solution aqueuse saturée en chlorure de sodium. La solution organique est séchée sur sulfate de magnésium anhydre et traitée au noir 3S, filtrée et concentrée à sec sous pression réduite (2,7 kPa) pour donner 0,115 g d'un solide huileux qui est purifié par chromatographie-flash [éluant : dichlorométhane / méthanol / acétonitrile (95/ 2,5 / 2,5 en volume)]. Après concentration des fractions sous pression réduite, on obtient 0,016 g de, 2-formylamino-4-{4-[3-(2-fluoro-5-trifluorométhyl-phényl)-uréido]-phényl}-1 H-pyrrole-3-carboxamide sous forme d'un solide crème fondant à 169°C. R.M.N. 1 H (400 MHz, (CD₃)₂SO, - δ en ppm) 5,60 (m étalé, 1 H) ; 6,41 (s large, 1 H) ; 7,07 (m étalé, 1 H) ; 7,31 (d, J = 8,5 Hz, 2H) ; 7,38 (m, 1 H) ; 7,48 (m partiellement masqué, 1 H) ; 7,51 (d, J = 8,5 Hz, 2H) ; 8,34 (s, 1 H) ; 8,61 (dd, J = 2,5 et 7,5 Hz, 1 H) ; 9,07 (s large, 1 H) ; 9,40 (s large, 1 H) ; 10,85 (m étalé, 1 H) ; 11,45 (s large, 1 H) .
Le 2-formylamino-4-(4-amino-phényl)-1H-pyrrole-3-carboxamide peut être préparé de la manière suivante :
   A une suspension de 0,030 g (0,0285 mmol) de palladium sur charbon à 10% dans 15 cm³ de méthanol, on ajoute à une température voisine de 25°C, 0,23 g (0,834 mmol) de 2-formylamino-4-(4-nitro-phényl)-1 H-pyrrole-3-carboxamide. Après 5 heures d'hydrogénation en autoclave sous 2 bars d'hydrogène, à une température voisine de 25°C, le mélange réactionnel est filtré, le catalyseur est rincé avec 3 fois 5 cm³ de méthanol puis le filtrat est concentré à sec sous pression réduite (2,7 kPa) pour donner 0,125 g de 2-formylamino-4-(4-amino-phényl)-1-H-pyrrole-3-carboxamide, sous forme d'un solide vert. R.M.N. 1H (400 MHz, (CD₃)₂SO, - δ en ppm) 5,13 (s large, 2H) ; 5,43 (m étalé, 1 H) ; 6,27 (d, J = 2,5 Hz, 1 H) ; 6,60 (d, J = 9,0 Hz, 2H) ; 6,80 (m étalé, 1 H) ; 6,99 (d, J = 9,0 Hz, 2H) ; 8,34 (d, J = 1,5 Hz, 1 H) ; 10,9 (s large, 1 H) ; 11,25 (s large, 1 H)
Le 2-formylamino-4-(4-nitro-phényl)-1H-pyrrole-3-carboxamide peut être préparé de la manière suivante :
   A une solution de 0,3 g (1,21 mmol) de 2-amino-4-(4-nitro-phényl)-1 H-pyrrole-3-carboxamide dans 5 cm³ d'éthanol absolu est additionné, à température proche de 25°C, une solution de 2 cm³ (52,9 mmol) d'acide formique dans 5 cm³ (52,9 mmol) d'anhydride acétique. Après 2 heures d'agitation à cette température, le milieu réactionnel est versé dans 100 cm³ d'eau. La suspension est alors filtrée. Le solide est essoré, séché pour donner 0,257 g de 2-formylamino-4-(4-nitro-phényl)-1H-pyrrole-3-carboxamide sous forme d'une poudre verte. R.M.N. 1 H (400 MHz, (CD₃)₂SO, - □ en ppm), de 6,22 à 8,53 (m très étalé, 2H) ; 6,78 (d, J = 3,0 Hz, 1H) ; 7,64 (d, J = 9,0 Hz, 2H) ; 8,19 (d, J = 9,0 Hz, 2H) ; 8,32 (d, J = 1,5 Hz, 1H) ; 10,55 (s large, 1 H) ; 11,6 (s large, 1 H).
Le 2-amino-4-(4-nitro-phényl)-1H-pyrrole-3-carboxamide est préparé comme décrit dans l'exemple 2 :

### Exemple 4

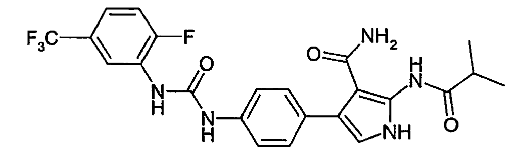

2-Isobutyrylamino-4-{4-[3-(2-fluoro-5-trifluorométhyl-phényl)-uréido]-phényl}-1 H-pyrrole-3-carboxamide :
   A 0,125 g (0,436 mmol) de 2-isobutyrylamino-4-(4-amino-phényl)-1H-pyrrole-3-carboxamide en solution dans 15 cm³ de tétrahydrofurane, on ajoute à une température voisine de 25°C, 0,076 cm³ (0,436 mmol) de 2-fluoro-5-trifluorométhyl-phényl isocyanate. Après 17 heures d'agitation à une température voisine de 25°C, le mélange réactionnel est concentré à sec sous pression réduite (2,7 kPa). Le résidu est alors dilué dans 40 cm³ d'acétate d'éthyle puis lavé avec 40 cm³ d'eau. La solution organique est séchée sur sulfate de magnésium anhydre, filtrée et concentrée à sec sous pression réduite (2,7 kPa) pour donner un résidu qui est cristallisé dans 8 cm³ d'un mélange cyclohexane / acétate d'éthyle (70 / 30 en volume). Après filtration et essorage, 0,096 g de 2-isobutyrylamino-4-{4-[3-(2-fluoro-5-trifluorométhyl-phényl)-uréido]-phényl}-1 H-pyrrole-3-carboxamide sous forme d'un solide crème fondant à 196°C. IR (KBr), 3472; 3384; 1667; 1594; 1546; 1443; 1340; 1313; 1198; 1167; 1120; 1070; 937 & 614 cm^{-1.} R.M.N. 1H (400 MHz, (CD₃)₂SO, - δ en ppm), 1,17 (d, J = 7,0 Hz, 6H) ; 2,61 (m, 1H) ; 5,62 (m étalé, 1 H) ; 6,40 (d, J = 2,5 Hz, 1 H) ; 7,00 (m étalé, 1 H) ; 7,30 (d, J = 9,0 Hz, 2H) ; 7,39 (m, 1 H) ; 7,49 (m partiellement masqué, 1 H) ; 7,51 (d, J = 9,0 Hz, 2H) ; 8,62 (dd, J = 2,5 et 7,5 Hz, 1 H) ; 8,99 (s large, 1 H) ; 9,32 (s large, 1H) ; 10,95 (s large, 1 H) ; 11,45 (s large, 1 H)
Le 2-isobutyrylamino-4-(4-amino-phényl)-1H-pyrrole-3-carboxamide peut être préparé de la manière suivante :
   A une suspension de 0,047 g (0,0446 mmol) de palladium sur charbon à 10% dans 25 cm³ de méthanol, on ajoute à une température voisine de 25°C, 0,33 g (1,04 mmol) de 2-isobutyrylamino-4-(4-nitro-phényl)-1H-pyrrole-3-carboxamide. Après 3,5 heures d'hydrogénation en autoclave sous 2 bars d'hydrogène, à une température voisine de 25°C, le mélange réactionnel est filtré, le catalyseur est rincé avec 3 fois 5 cm³ de méthanol puis le filtrat est concentré à sec sous pression réduite (2,7 kPa) pour donner 0,22 g d'un résidu qui est purifié par chromatographie-flash [éluant : dichlorométhane /méthanol / acétonitrile (96/ 2 / 2 en volume)]. Après concentration des fractions sous pression réduite, on obtient 0,135 g de 2-isobutyrylamino-4-(4-amino-phényl)-1-H-pyrrole-3-carboxamide, sous forme d'un solide marron. R.M.N. 1H (400 MHz, (CD₃)₂SO, -δ en ppm) 1,16 (d, J = 7,0 Hz, 6H) ; 2,60 (m, 1 H) ; 5,15 (s large, 2H) ; 5,45 (m étalé, 1 H) ; 6,24 (d, J = 2,0 Hz, 1 H) ; 6,60 (d, J = 9,0 Hz, 2H) ; 6,94 (m étalé, 1 H) ; 6,98 (d, J = 9,0 Hz, 2H) ; 11,05 (s large, 1 H) ; 11,3 (s large, 1 H).
Le 2-isobutyrylamino-4-(4-nitro-phényl)-1H-pyrrole-3-carboxamide peut être préparé de la manière suivante :
   A une solution de 0,23 g (0,93 mmol) de 2-amino-4-(4-nitro-phény)-1H-pyrrole-3-carboxamide dans 15 cm³ de tétrahydrofurane est additionné, à température proche de 25°C, 0,260 cm³ (1,86 mmol) de triéthylamine et 0,098 cm³ (0,93 mmol) de chlorure d'isobutyryle. Après 16 heures d'agitation à cette température, le milieu réactionnel est concentré à sec sous pression réduite (2,7 kPa) pour donner un résidu qui est agité dans 60 cm³ d'eau puis extrait avec 2 fois 50 cm³ d'acétate d'éthyle. La phase organique est séchée sur sulfate de magnésium anhydre, filtrée et concentrée à sec sous pression réduite (2,7 kPa) pour donner 0,35 g de 2-isobutyrylamino-4-(4-nitro-phényl)-1 H-pyrrole-3-carboxamide sous forme d'une poudre verte. R.M.N. 1 H (400 MHz, (CD₃)₂SO, - □ en ppm) 15 (d, J = 7,0 Hz, 6H) ; 2,64 (m, 1 H) ; de 6,50 à 8,50 (m très étalé, 2H) ; 6,79 (d, J = 2,5 Hz, 1H); 7,64 (d, J = 9,0 Hz, 2H) ; 8,19 (d, J = 9,0 Hz, 2H) ; 10,45 (s large, 1H); 11,65 (s large, 1H).
Le 2-amino-4-(4-nitro-phényl)-1 H-pyrrole-3-carboxamide est préparé comme décrit dans l'exemple 2.

### Exemple 5

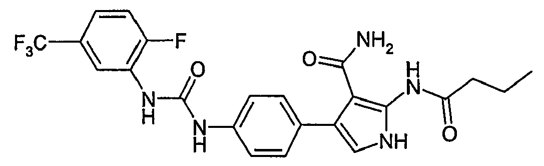

2-Butyrylamino-4-{4-[3-(2-fluoro-5-trifluorométhyl-phényl)-uréido]-phényl}-1H-pyrrole-3-carboxamide :
   A 0,207 g (0,610 mmol) de 2-butyrylamino-4-(4-amino-phényl)-1H-pyrrole-3-carboxamide en solution dans 20 cm³ de tétrahydrofurane, on ajoute à une température voisine de 25°C, 0,096 cm³ (0,671 mmol) de 2-fluoro-5-trifluorométhyl-phényl isocyanate. Après 48 heures d'agitation à une température voisine de 25°C, le mélange réactionnel est concentré à sec sous pression réduite (2,7 kPa). Le résidu est alors dilué dans 40 cm³ d'acétate d'éthyle puis lavé avec 2 fois 30 cm³ d'eau et 30 cm³ d'une solution aqueuse saturée en chlorure de sodium. La solution organique est séchée sur sulfate de magnésium anhydre, filtrée et concentrée à sec sous pression réduite (2,7 kPa) pour donner un résidu qui est purifié par chromatographie-flash [éluant : dichlorométhane / méthanol / acétonitrile (97 / 1,5 / 1,5 en volume)]. Après concentration des fractions sous pression réduite, on obtient 0,093 g de 2-butyrylamino-4-{4-[3-(2-fluoro-5-trifluorométhyl-phényl)-uréido]-phényl}-1 H-pyrrole-3-carboxamide sous forme d'un solide crème fondant à 219°C. IR (KBr), 3470; 3387; 1717; 1626; 1593; 1546; 1443; 1341; 1315; 1264; 1193; 1168; 1126; 1070; 820; 614 cm⁻¹. R.M.N. 1H (400 MHz, (CD₃)₂SO, -δ en ppm) 0,95 (t, J = 7,5 Hz, 3H) ; 1,64 (m, 2H) ; 2,38 (t, J = 7,5 Hz, 2H) ; 5,60 (m étalé, 1 H) ; 6,39 (d, J = 2,5 Hz, 1 H) ; 7,05 (m étalé, 1H) ; 7,30 (d, J = 9,0 Hz, 2H) ; 7,38 (m, 1H) ; 7,49 (m partiellement masqué, 1H) ; 7,51 (d, J = 9,0 Hz, 2H) ; 8,61 (dd, J = 2,5 et 7,5 Hz, 1 H) ; 9,05 (s large, 1H) ; 9,38 (s large, 1 H) ; 10,8 (s large, 1H) ; 11,4 (s large, 1 H).
Le 2-butyrylamino-4-(4-amino-phényl)-1H-pyrrole-3-carboxamide peut être préparé de la manière suivante :
   A une suspension de 0,068 g (0,064 mmol) de palladium sur charbon à 10% dans 20 cm³ de méthanol, on ajoute à une température voisine de 25°C, 0,195 g (0,61 mmol) de 2-butyrylamino-4-(4-nitro-phényl)-1H-pyrrole-3-carboxamide. Après 5 heures d'hydrogénation en autoclave sous 2 bars d'hydrogène, à une température voisine de 25°C, le mélange réactionnel est filtré, le catalyseur est rincé avec 2 fois 10 cm³ de méthanol puis le filtrat est concentré à sec sous pression réduite (2,7 kPa) pour donner 0,207 g de 2-butyrylamino-4-(4-amino-phényl)-1-H-pyrrole-3-carboxamide, sous forme d'un solide crème. R.M.N. 1H (400 MHz, (CD₃)₂SO, -δ en ppm) 0,94 (t, J = 7,5 Hz, 3H) ; 1,63 (m, 2H) ; 2,37 (t, J = 7,5 Hz, 2H) ; 5,11 (s large, 2H) ; de 6,05 à 8,45 (m très étalé, 2H) ; 6,24 (d, J = 2,5 Hz, 1 H) ; 6,60 (d, J = 8,5 Hz, 2H) ; 6,99 (d, J = 8,5 Hz, 2H) ; 10,8 (s large, 1 H) ; 11,3 (s large, 1 H) .
Le 2-butyrylamino-4-(4-nitro-phényl)-1H-pyrrole-3-carboxamide peut être préparé de la manière suivante :
   A une solution de 0,20 g (0,81 mmol) de 2-amino-4-(4-nitro-phényl)-1H-pyrrole-3-carboxamide dans 25 cm³ de tétrahydrofurane est additionné, à température proche de 25°C, 0,227 cm³ (1,62 mmol) de triéthylamine et 0,085 cm³ (0,81 mmol) de chlorure de butyryle. Après 16 heures d'agitation à cette température, le milieu réactionnel est concentré à sec sous pression réduite (2,7 kPa) pour donner un résidu qui est agité dans 60 cm³ d'acétate d'éthyle puis lavé avec 3 fois 20 cm³ d'eau. La phase organique est séchée sur sulfate de magnésium anhydre, filtrée et concentrée à sec sous pression réduite (2,7 kPa) pour donner 0,221 g de 2-butyrylamino-4-(4-nitro-phényl)-1 H-pyrrole-3-carboxamide sous forme d'une poudre marron. R.M.N. 1 H (400 MHz, (CD₃)₂SO, - δ en ppm) 0,94 (t, J = 7,5 Hz, 3H) ; 1,63 (m, 2H) ; 2,36 (t, J = 7,5 Hz, 2H) ; de 6,20 à 8,50 (m très étalé, 2H) ; 6,79 (d, J = 3,0 Hz, 1 H) ; 7,64 (d, J = 9,0 Hz, 2H) ; 8,18 (d, J = 9,0 Hz, 2H) ; 10,3 (s large, 1 H) ; 11,6 (s large, 1 H)
Le 2-amino-4-(4-nitro-phényl)-1 H-pyrrole-3-carboxamide est préparé comme décrit dans l'exemple 2.

### Exemple 6

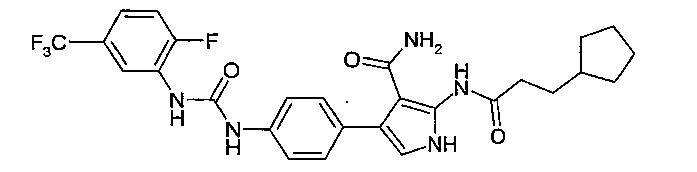

2-(3-Cyclopentyl-propionylamino)-4-{4-[3-(2-fluoro-5-trifluorométhyl-phényl)-uréido]-phényl}-1H-pyrrole-3-carboxamide :
   A 0,170 g (0,50 mmol) de 2-(3-cyclopentyl-propionylamino)-4-(4-aminophényl)-1 H-pyrrole-3-carboxamide en solution dans 20 cm³ de tétrahydrofurane, on ajoute à une température voisine de 25°C, 0,072 cm³ (0,50 mmol) de 2-fluoro-5-trifluorométhyl-phényl isocyanate. Après 48 heures d'agitation à une température voisine de 25°C, le mélange réactionnel est concentré à sec sous pression réduite (2,7 kPa). Le résidu est alors dilué dans 50 cm³ d'acétate d'éthyle puis lavé avec 50 cm³ d'eau et 50 cm³ d'une solution aqueuse saturée en chlorure de sodium. La solution organique est séchée sur sulfate de magnésium anhydre, filtrée et concentrée à sec sous pression réduite (2,7 kPa) pour donner un résidu qui est purifié par chromatographie-flash [éluant : dichlorométhane / méthanol / acétonitrile (95 /2,5 / 2,5 en volume)]. Après concentration des fractions sous pression réduite, on obtient 0,048 g de 2-(3-cyclopentyl-propionylamino)-4-{4-[3-(2-fluoro-5-trifluorométhyl-phényl)-uréido]-phényl}-1 H-pyrrole-3-carboxamide sous forme d'une poudre jaune fondant à 222°C. IR (KBr), 3470; 3389; 1717; 1633; 1594; 1546; 1443; 1341; 1312; 1194; 1167; 1119; 1070 & 614 cm⁻¹. R.M.N. 1H (400 MHz, (CD₃)₂SO, - δ en ppm) 1,11 (m, 2H) ; de 1,42 à 1,68 (m, 6H) ; de 1,71 à 1,85 (m, 3H) ; 2,40 (t, J = 8,0 Hz, 2H) ; 5,56 (m étalé, 1 H) ; 6,39 (d, J = 2,5 Hz, 1H) ; 7,09 (m étalé, 1H) ; 7,30 (d, J = 9,0 Hz, 2H) ; 7,38 (m, 1 H) ; 7,49 (m partiellement masqué, 1H) ; 7,51 (d, J = 9,0 Hz, 2H) ; 8,62 (dd, J = 2,5 et 7,5 Hz, 1 H) ; 9,00 (s large, 1 H) ; 9,32 (s large, 1 H) ; 10,8 (s large, 1 H) ; 11,4 (s large, 1 H).
Le 2-(3-cyclopentyl-propionylamino)-4-(4-amino-phényl)-1 H-pyrrole-3-carboxamide peut être préparé de la manière suivante :
   A une suspension de 0,055 g (0,0051 mmol) de palladium sur charbon à 10% dans 25 cm³ de méthanol, on ajoute à une température voisine de 25°C, 0,210 g (0,56 mmol) de 2-(3-cyclopentyl-propionylamino)-4-(4-nitro-phényl)-1 H-pyrrole-3-carboxamide. Après 5 heures d'hydrogénation en autoclave sous 2 bars d'hydrogène, à une température voisine de 25°C, le mélange réactionnel est filtré, le catalyseur est rincé avec 2 fois 10 cm³ de méthanol puis le filtrat est concentré à sec sous pression réduite (2,7 kPa) pour donner 0,151 g de 2-(3-Cyclopentyl-propionylamino)-4-(4-amino-phényl)-1-H-pyrrole-3-carboxamide, sous forme d'une poudre marron. R.M.N. 1 H (400 MHz, (CD₃)₂SO, - δ en ppm) 1,09 (m, 2H) ; de 1,39 à 1,84 (m, 9H) ; 2,39 (t, J = 8,0 Hz, 2H) ; 5,14 (s large, 2H) ; de 6,20 à 7,50 (m très étalé, 2H) ; 6,23 (d, J = 2,5 Hz, 1 H) ; 6,59 (d, J = 8,5 Hz, 2H) ; 6,99 (d, J = 8,5 Hz, 2H) ; 10,9 (s large, 1 H) ; 11,3 (s large, 1 H) .
Le 2-(3-cyclopentyl-propionylamino)-4-(4-nitro-phényl)-1H-pyrrole-3-carboxamide peut être préparé de la manière suivante
   A une solution de 0,15 g (0,61 mmol) de 2-amino-4-(4-nitro-phényl)-1H-pyrrole-3-carboxamide dans 25 cm³ de tétrahydrofurane est additionné, à température proche de 25°C, 0,171 cm³ (1,22 mmol) de triéthylamine et 0,098 mg (0,61 mmol) de chlorure de 3-cyclopentyl-propionyle. Après 16 heures d'agitation à cette température, le milieu réactionnel est concentré à sec sous pression réduite (2,7 kPa) pour donner un résidu qui est agité dans 40 cm³ d'eau puis extrait avec 3 fois 40 cm³ d'acétate d'éthyle. La phase organique est lavée avec 80 cm³ d'une solution aqueuse saturée en chlorure de sodium, séchée sur sulfate de magnésium anhydre, filtrée et concentrée à sec sous pression réduite (2,7 kPa) pour donner 0,218 g de 2-(3-Cyclopentyl-propionylamino)-4-(4-nitro-phényl)-1 H-pyrrole-3-carboxamide sous forme d'une poudre verte. R.M.N. 1 H (400 MHz, (CD₃)₂SO, - δ en ppm) 1,11 (m, 2H) ; de 1,42 à 1,65 (m, 6H) ; de 1,69 à 1,86 (m, 3H) ; 2,39 (t, J = 8,0 Hz, 2H) ; de 6,17 à 8,50 (m très étalé, 2H) ; 6,79 (d, J = 2,5 Hz, 1 H) ; 7,63 (d, J = 9,0 Hz, 2H) ; 8,18 (d, J = 9,0 Hz, 2H) ; 10,35 (s large, 1 H) ; 11,6 (s large, 1 H) Le 2-amino-4-(4-nitro-phényl)-1 H-pyrrole-3-carboxamide est préparé comme décrit dans l'exemple 2.

### Exemple 7

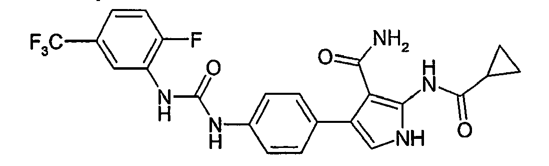

2-(Cyclopropylcarbonylamino)-4-{4-[3-(2-fluoro-5-trifluorométhyl-phényl)-uréido]-phényl}-1 H-pyrrole-3-carboxamide
   Le 2-(cyclopropylcarbonylamino)-4-{4-[3-(2-fluoro-5-trifluorométhyl-phényl)-uréido]-phényl}-1 H-pyrrole-3-carboxamide peut être préparé comme décrit dans l'exemple 4 à partir de 2-amino-4-(4-nitro-phényl)-1H-pyrrole-3-carboxamide et du chlorure de cyclopropylcarbonyle. ES+: m/z = 490 (MH⁺).

### Exemple 8

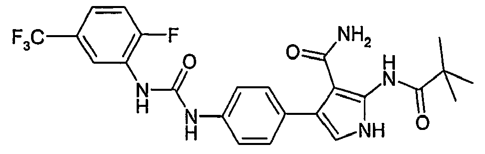

2-Pivaloylamino-4-{4-[3-(2-fluoro-5-trifluorométhyl-phényl)-uréido]-phényl}-1H-pyrrole-3-carboxamide
   Le 2-pivaloylamino-4-{4-[3-(2-fluoro-5-trifluorométhyl-phényl)-uréido]-phényl}-1 H-pyrrole-3-carboxamide peut être préparé comme décrit dans l'exemple 4 à partir de 2-amino-4-(4-nitro-phényl)-1 H-pyrrole-3-carboxamide et du chlorure de pivaloyle ES+: m/z = 506 (MH⁺).

### Exemple 9

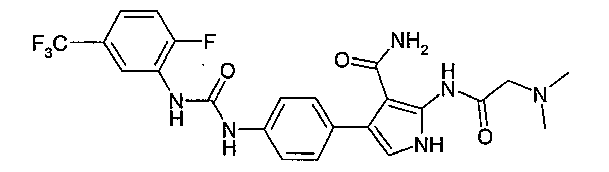

2-(2-Diméthylamino-acetylamino)-4-{4-[3-(2-fluoro-5-trifluorométhyl-phényl)-uréido]-phényl}-1H-pyrrole-3-carboxamide
   Le 2-(2-diméthylamino-acetylamino)-4-{4-[3-(2-fluoro-5-trifluorométhyl-phényl)-uréido]-phényl}-1H-pyrrole-3-carboxamide peut être préparé comme décrit dans l'exemple 4 à partir de 2-amino-4-(4-nitro-phényl)-1 H-pyrrole-3-carboxamide et du chlorure d'acide de la diméthylglycine ES+: m/z = 507 (MH⁺).

### Exemple 10

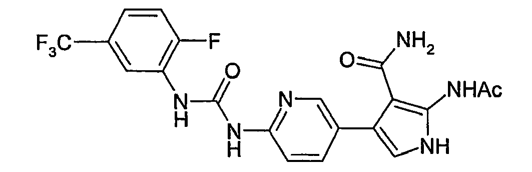

2-Acétylamino-4-{6-[3-(2-fluoro-5-trifluorométhyl-phényl)-uréido]-pyridin-3-yl}-1 H-pyrrole-3-carboxamide :
   A 0,11 g (0,424 mmol) de 2-acétylamino-4-(6-amino-pyridin-3-yl)-1 H-pyrrole-3-carboxamide en solution dans 20 cm³ de tétrahydrofurane, on ajoute à une température voisine de 23°C et sous atmosphère d'argon, 0,068 cm³ (0,466 mmol) de 2-fluoro-5-trifluorométhyl-phényl isocyanate. Après 1 heure d'agitation à une température voisine de 20°C, 0,059 cm³ (0,424 mmol) de triéthylamine sont additionnés au milieu. Le mélange réactionnel est alors agité 18 heures à cette température puis concentré à sec sous pression réduite (2,7 kPa). Le résidu est purifié par chromatographie-flash [éluant : gradient dichlorométhane / méthanol (95 / 5 en volume) et acétate d'éthyle pur]. Après concentration des fractions sous pression réduite, on obtient 0,013 g de, 2-acétylamino-4-{6-[3-(2-fluoro-5-trifluorométhyl-phényl)uréido]-pyridin-3-yl}-1H-pyrrole-3-carboxamide sous forme d'un solide blanc. ES+: m/z = 466 (MH⁺).
Le 2-acétylamino-4-(6-amino-pyridin-3-yl)-1H-pyrrole-3-carboxamide peut être préparé de la manière suivante :
   A une suspension de 0,015 g (0,014 mmol) de palladium sur charbon à 10% dans 20 cm³ de méthanol, on ajoute à une température voisine de 25°C, 0,15 g (0,519 mmol) de 2-acétylamino-4-(6-nitro-pyridin-3-yl)-1H-pyrrole-3-carboxamide. Après 2 heures d'hydrogénation en autoclave sous 2 bars d'hydrogène, à une température voisine de 30°C, le mélange réactionnel est filtré, le catalyseur est rincé avec 2 fois 2 cm³ d'éther éthylique. Après essorage et séchage, on obtient 0,11 g de 2-acétylamino-4-(6-amino-pyridin-3-yl)-1-H-pyrrole-3-carboxamide, sous forme d'un solide marron. R.M.N. 1 H (400 MHz, (CD₃)₂SO, - δ en ppm) 2,13 (s, 3H) ; 6,01 (s large, 2H) ; de 5,50 à 8,85 (m très étalé, 2H) ; 6,35 (d, J = 2,5 Hz, 1 H) ; 6,49 (d, J = 8,5 Hz, 1H) ; 7,36 (dd, J = 2,5 et 8,5 Hz, 1 H) ; 7,87 (d, J = 2,5 Hz, 1 H) ; 10,65 (s, 1H) ; 11,35 (s large, 1 H).
Le 2-acétylamino-4-(6-nitro-pyridin-3-yl)-1H-pyrrole-3-carboxamide peut être préparé de la manière suivante :
   A une solution de 0,20 g (0,809 mmol) de 2-amino-4-(6-nitro-pyridin-3-yl)-1H-pyrrole-3-carboxamide dans 40 cm³ de tétrahydrofurane est additionné, à température proche de 20°C sous atmosphère d'argon, 0,226 cm³ (1,62 mmol) de triéthylamine et 0,058 cm³ (0,809 mmol) de chlorure d'acétyle. Après 3 heures d'agitation à cette température, le milieu réactionnel est concentré à sec sous pression réduite (2,7 kPa) pour donner un résidu qui est dilué dans 200 cm³ d'acétate d'éthyle puis lavé avec 50 cm³ d'eau et 50 cm³ d'une solution aqueuse saturée en chlorure de sodium, séché sur sulfate de magnésium anhydre, filtré et concentré à sec sous pression réduite (2,7 kPa) pour donner 0,15 g de 2-acétylamino-4-(6-nitro-pyridin-3-yl)-1H-pyrrole-3-carboxamide sous forme d'une poudre verte. R.M.N. 1H (400 MHz, (CD₃)₂SO, - δ en ppm) 2,11 (s, 3H) ; 6,90 (m étalé, 2H) ; 6,95 (d, J = 3,0 Hz, 1 H) ; 8,13 (dd, J = 2,5 et 8,5 Hz, 1 H) ; 8,27 (d, J = 8,5 Hz, 1 H) ; 8,65 (d, J = 2,5 Hz, 1 H) ; 10,2 (s, 1 H) ; 11,7 (m large, 1 H).
Le 2-amino-4-(6-nitro-3-pyridyl)-1 H-pyrrole-3-carboxamide peut être préparé de la manière suivante :
   4,5 cm³ d'acide sulfurique concentré sont additionnées sur 0,17 g (0,742 mmol) de 2-amino-4-(6-nitro-3-pyridin-3-yl)-1H-pyrrole-3-carbonitrile à une température voisine de 5°C. Le mélange est chauffé à une température voisine de 85°C pendant 1 heure sous atmosphère d'argon. Après avoir refroidi le mélange réactionnel à 5°C, on verse celui-ci sur de la glace pilée et 30 cm³ d'eau. Cette solution est versée sur une solution de 300 cm³ de tétrahydrofurane et 15 cm³ de pyridine. Après 5 minutes d'agitation la phase organique est lavée avec 30 cm³ d'une solution aqueuse saturée en chlorure de sodium puis séchée sur sulfate de magnésium anhydre, filtrée et concentrée à sec sous pression réduite (2,7 kPa) pour donner 0,20 g de 2-amino-4-(6-nitro-pyridin-3-yl)-1H-pyrrole-3-carboxamide, sous forme d'un solide marron. ES+: m/z = 248 (MH⁺).
Le 2-amino-4-(6-nitro-pyridin-3-yl)-1 H-pyrrole-3-carbonitrile peut être préparé de la manière suivante :
   A 0,050 g (0,224 mmol) de N-[2-(6-nitro-pyridin-3-yl)-2-oxo-éthyl]-acétamide en solution dans 5 cm³ de méthanol, on ajoute à une température voisine de 20°C, sous atmosphère d'argon, 0,022 g (0,336 mmol) de malononitrile. Le milieu réactionnel est refroidi à une température voisine de 0°C puis 0,1 cm³ d'une solution aqueuse à 50% d'hydroxyde de potassium est ajoutée. Après 15 minutes d'agitation à une température voisine de 20°C puis 1,15 heures d'agitation à une température voisine de 65°C, le mélange réactionnel est concentré à sec sous pression réduite (2,7 kPa) puis dilué dans 50 cm³ d'acétate d'éthyle. La phase organique est lavée avec 10 cm³ d'eau et 10 cm³ d'une solution aqueuse saturée en chlorure de sodium puis séchée sur sulfate de magnésium anhydre, filtrée et concentrée à sec sous pression réduite (2,7 kPa) pour donner 0,043 g de 2-amino-4-(6-nitro-pyridin-3-yl)-1 H-pyrrole-3-carbonitrile sous forme d'un solide brun. ES+: m/z = 230 (MH⁺).
Le N-[2-(6-nitro-pyridin-3-yl)-2-oxo-éthyl]-acétamide peut être préparé de la manière suivante :
   A 1,05 g (4,166 mmol) de 2-amino-1-(6-nitro-pyridin-3-yl)-éthanone en solution dans 25 cm³ d'eau, on ajoute à une température voisine de 5°C, 1,575 cm³ (16,66 mmol) d'anhydride acétique puis 1,367 g (16,66 mmol) d'acétate de sodium en solution dans 3 cm³ d'eau. Après 3 heures d'agitation à 5°C, le mélange réactionnel est extrait par 3 fois 10 cm³ d'acétate d'éthyle. Toutes les phases organiques sont réunies, lavées avec 30 cm³ d'une solution aqueuse saturée en chlorure de sodium puis séchées sur sulfate de magnésium anhydre, filtrées et concentrées à sec sous pression réduite (2,7 kPa) pour donner 0,618 g de N-[2-(6-nitro-pyridin-3-yl)-2-oxo-éthyl]-acétamide, sous forme d'un solide jaune. R.M.N. 1 H (400 MHz, (CD₃)₂SO, - δ en ppm) 1,90 (s, 3H) ; 4,66 (d, J = 4,5 Hz, 2H) ; 8,37 (t large, J = 4,5 Hz, 1H) ; 8,43 (d, J = 8,5 Hz, 1 H) ; 8,68 (dd, J = 2,0 et 8,5 Hz, 1 H) ; 9,16 (d, J = 2,0 Hz, 1 H).
La 2-amino-1-(6-nitro-pyridin-3-yl)-éthanone peut être préparée de la manière suivante :
   A une solution de 0,975 g (6,954 mmol) d'héxaméthylène tétramine dans 10 cm³ de chlorobenzène on ajoute à une température voisine de 20°C une solution de 1,549 g (6,322 mmol) de 2-bromo-1-(6-nitro-pyridin-3-yl)-éthanone dans 25 cm³ de chlorobenzène. Après 1 heure d'agitation à cette température, la suspension est chauffée 18 heures à 50°C. Le milieu réactionnel est alors refroidit à 5 °C puis dilué avec 200 cm³ d'éther éthylique. Le précipité ainsi obtenu est filtré et lavé avec 3 fois 50 cm³ d'éther éthylique. Le sel d'ammonium résultant est agité dans 20 cm³ d'éthanol puis 8 cm³ d'acide chlorhydrique à 37% sont additionnés à une température voisine 20°C. La solution est alors agitée 16 heures à cette température. Le précipité formé est filtré, lavé avec 3 fois 50 cm3 d'eau, essoré et séché pour donner 1,05 g de 2-amino-1-(6-nitro-pyridin-3-yl)-éthanone sous forme d'une poudre crème. ES+: m/z = 182 (MH⁺).
La 2-bromo-1-(6-nitro-pyridin-3-yl)-éthanone peut être préparée de la manière suivante :
   A une solution de 3,4 g (20,46 mmol) de 1-(6-nitro-pyridin-3-yl)-éthanone dans 60 cm³ de tétrahydrofurane sont ajoutés 7,28 g (40,92 mmol) de N-bromosuccinimide à une température voisine de 20°C. Après 36 heures de chauffage au reflux, le mélange réactionnel est concentré à sec sous pression réduite (2,7 kPa) puis purifié par chromatographie-flash [éluant : dichlorométhane pur]. Après concentration des fractions sous pression réduite, on obtient 1,7 g de 2-bromo-1-(6-nitro-pyridin-3-yl)-éthanone sous forme d'une poudre blanche. ES+: m/z = 246 (MH⁺).
Le 1-(6-nitro-pyridin-3-yl)-éthanone peut être préparé de la manière suivante :
   A une solution 1,044 g (3,98 mmol) de triphénylphosphine dans 10 cm³ de toluène on ajoute à une température voisine de 20°C sous atmosphère d'argon, 1,14 g (1,99 mmol) de bis (dibenzylidène acétone) palladium et 10,1 g (49,75 mmol) de 5-bromo-2-nitropyridine. Après 15 minutes d'agitation à cette température, on ajoute une solution 16,9 cm³ (49,75 mmol) de 1-éthoxyvinyltributylétain dans 60 cm³ de toluène. Après 15 heures de chauffage au reflux, le milieu réactionnel est refroidi à 20 °C puis versé dans 500 cm³ d'une solution d'acide chlorhydique 1 N et agité 16 heures à cette température. Le milieu est alors extrait avec 3 fois 150 cm³ d'acétate d'éthyle. Les phases organiques sont rassemblées puis lavées avec 300 cm³ d'eau, séchées sur sulfate de magnésium anhydre, filtrées et concentrées à sec sous pression réduite (2,7 kPa) pour donner un résidu qui est purifié par chromatographie-flash [éluant : dichlorométhane pur]. Après concentration des fractions sous pression réduite, on obtient 3,4 g 1-(6-nitro-pyridin-3-yl)-éthanone. R.M.N. 1 H (400 MHz, (CD₃)₂SO, - δ en ppm) 2,71 (s, 3H) ; 8,42 (d, J = 8,5 Hz, 1 H) ; 8,66 (dd, J = 2,5 et 8,5 Hz, 1 H) ; 9,15 (d, J =2,5 Hz, 1 H).

### Exemple 11

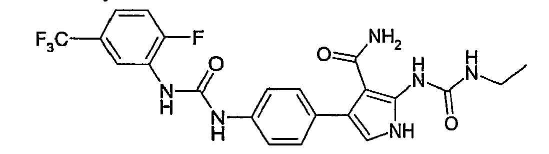

2-(3-Éthyl-uréido)-4-{4-[3-(2-fluoro-5-trifluorométhyl-phényl)-uréido]-phényl}-1 H-pyrrole-3-carboxamide
   A 0,056 g (0,194 mmol) de 4-(4-amino-phényl)-2-(3-éthyl-uréido)-1H-pyrrole-3-carboxamide en solution dans 20 cm³ de tétrahydrofurane, on ajoute à une température voisine de 25°C, 0,031 cm³ (0,0213 mmol) de 2-fluoro-5-trifluorométhyl-phényl isocyanate. Après 16 heures d'agitation à une température voisine de 60°C, le mélange réactionnel est concentré à sec sous pression réduite (2,7 kPa). Le résidu est alors dilué dans 40 cm³ d'acétate d'éthyle puis lavé avec 2 fois 30 cm³ d'eau. La solution organique est séchée sur sulfate de magnésium anhydre, filtrée et concentrée à sec sous pression réduite (2,7 kPa) pour donner un résidu qui est purifié par chromatographie-flash [éluant : dichlorométhane / méthanol / acétonitrile (95 /2,5 / 2,5 en volume)]. Après concentration des fractions sous pression réduite, on obtient 0,021 g de 2-(3-éthyl-uréido)-4-{4-[3-(2-fluoro-5-trifluorométhyl-phényl)-uréido]-phényl}-1 H-pyrrole-3-carboxamide sous forme d'un solide jaune. ES+: m/z = 494 (MH⁺).
Le 4-(4-amino-phényl)-2-(3-éthyl-uréido)-1 H-pyrrole-3-carboxamide peut être préparé de la manière suivante :
   A une suspension de 0,067 g (0,0636 mmol) de palladium sur charbon à 10% dans 15 cm³ de méthanol, on ajoute à une température voisine de 25°C, 0,115 g (0,362 mmol) de 4-(4-nitro-phényl)-2-(3-éthyl-uréido)-1H-pyrrole-3-carboxamide. Après 2,5 heures d'hydrogénation en autoclave sous 2 bars d'hydrogène, à une température voisine de 25°C, le mélange réactionnel est filtré, le catalyseur est rincé avec 3 fois 5 cm³ de méthanol puis le filtrat est concentré à sec sous pression réduite (2,7 kPa) pour donner 0,056 g de 4-(4-amino-phényl)-2-(3-éthyl-uréido)-1 H-pyrrole-3-carboxamide, sous forme d'un solide orange; ES+: m/z = 289 (MH⁺).
Le 4-(4-nitro-phényl)-2-(3-éthyl-uréido)-1H-pyrrole-3-carboxamide peut être préparé de la manière suivante :
   A une solution de 0,2 g (0,81 mmol) de 2-amino-4-(4-nitro-phényl)-1 H-pyrrole-3-carboxamide dans 15 cm³ de tétrahydrofurane est additionné, à température proche de 25°C, 0,074 cm³ (0,891 mmol) de d'éthylisocyanate et 0,005 mg (0,040 mmol) de diméthylaminopyridine. Après 16 heures d'agitation une température proche de 60°C, le milieu réactionnel est concentré à sec sous pression réduite (2,7 kPa) pour donner un résidu qui est agité dans 100 cm³ d'eau puis extrait avec 3 fois 50 cm³ d'acétate d'éthyle. La phase organique est séchée sur sulfate de magnésium anhydre, filtrée et concentrée à sec sous pression réduite (2,7 kPa) pour donner un résidu qui est purifié par chromatographie-flash [éluant : dichlorométhane / méthanol /acétonitrile (95 / 2,5 / 2,5 en volume)]. Après concentration des fractions sous pression réduite, on obtient 0,120 g 4-(4-nitro-phényl)-2-(3-éthyl-uréido)-1H-pyrrole-3-carboxamide de sous forme d'une poudre jaune. ES+: m/z = 318 (MH⁺).
Le 2-amino-4-(4-nitro-phényl)-1H-pyrrole-3-carboxamide est préparé comme décrit dans l'exemple 2.

### Exemple 12

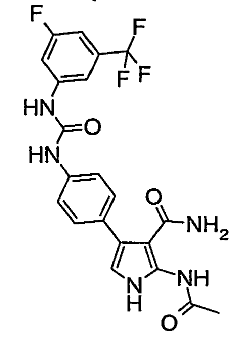

2-acétylamino-4-{4-[3-(3-fluoro-5-trifluorométhyl-phényl)-uréido]-phényl}-1 H-pyrrole-3-carboxamide
   0,061 cm³ (0,426 mmol) de 3-fluoro-5-trifluorométhyl-phénylisocyanate est ajouté à température ambiante à une suspension de 100 mg (0,387 mmol) de 2-acétylamino-4-(4-amino-phényl)-1 H-pyrrole-3-carboxamide dans 5 cm³ de tétrahydrofurane anhydre. Le milieu réactionnel est agité à température ambiante pendant 24 heures, refroidi dans un bain eau-glace puis filtré sur fritté. Le solide recueilli est lavé avec un peu de dichlorométhane et de cyclohexane puis séché sous vide. On obtient 75 mg de 2-acétylamino-4-{4-[3-(3-fluoro-5-trifluorométhyl-phényl)-uréido]-phényl}-1H-pyrrole-3-carboxamide sous forme d'un solide beige.
   LCMS (méthode A) : m/z = 464 : [M+H]⁺ ; m/z = 447 : [M+H]⁺ - NH₃ (pic de base)
   m/z = 462 : [M-H]⁻
   Temps de rétention (min.) = 3,97
Le 2-acétylamino-4-(4-amino-phényl)-1 H-pyrrole-3-carboxamide peut être préparé de la manière suivante :
   0,15 g de palladium sur charbon (10%) est ajouté à une suspension de 1,36 g (4,72 mmol) de 2-acétylamino-4-(4-nitro-phényl)-1 H-pyrrole-3-carboxamide dans 200 cm³ de méthanol La réaction est hydrogénée sous 2 bars à 30°C pendant 6 heures puis le milieu réactionnel est filtré sur célite, lavé avec du méthanol. Le filtrat est évaporé sous pression réduite et 1,14 g de 2-acétylamino-4-(4-amino-phényl)-1H-pyrrole-3-carboxamide sous forme de solide marron est obtenu.
   R.M.N. ¹H (300 MHz, (CD₃)₂SO d6 - δ en ppm) : 2,13 (s, 3H) ; 5,13 (s, 2H) ; 5,45 (m étalé, 1 H) ; 6,24 (d, J = 2,5 Hz, 1 H) ; 6,59 (d, J = 8,5 Hz, 2H) ; de 6,90 à 7,10 (m étalé, 1 H) ; 6,99 (d, J = 8,5 Hz, 2H) ; 10,8 (s, 1 H) ; 11,25 (s large, 1 H).
   IE : m/z = 258 : [M⁺˙] (pic de base) ; m/z = 241 : [M+H]⁺ - NH₃ ; m/z = 199 : 241-COCH₃
Le 2-acétylamino-4-(4-nitro-phényl)-1H-pyrrole-3-carboxamide peut être préparé de la manière suivante :
   0,851 cm³ (11,960 mmol) de chlorure d'acétyle est ajouté à température ambiante à une suspension de 2,16 g (8,77 mmol) de 2-amino-4-(4-nitro-phényl)-1H-pyrrole-3-carboxamide dans 200 cm³ de tétrahydrofurane sec sous argon. Le mélange est refroidi dans un bain eau - glace puis 3,180 cm³ (22,82 mmol) de triéthylamine sont ajoutés lentement à 0°C. La réaction est agitée à 0°C pendant 15 minutes puis à température ambiante pendant 3 heures. Le milieu est repris dans de l'acétate d'éthyle puis la phase organique est lavée à l'eau puis séchée sur sulfate de magnésium, filtrée et les solvants évaporés sous pression réduite. Le produit brut est purifié par chromatographie-flash [éluant: dichlorométhane / méthanol (99/1 en volume)]. Après concentration sous pression réduite des fractions contenant le produit attendu, on obtient 1,23 g de 2-acétylamino-4-(4-nitro-phényl)-1H-pyrrole-3-carboxamide sous forme d'un solide marron.
   R.M.N. ¹H (400 MHz, (CD₃)₂SO d6 - δ en ppm) : 2,11 (s, 3H) ; de 6,50 à 7,20 (m très étalé, 2H) ; 6,80 (s, 1 H) ; 7,63 (d, J = 9,0 Hz, 2H) ; 8,18 (d, J = 9,0 Hz, 2H) ; 10,3 (s large, 1 H) ; 11,6 (s large, 1 H).
   LCMS (méthode A) : m/z = 287 : [M-H]⁻
   Temps de rétention (min.) = 2,90
Le 2-amino-4-(4-nitro-phényl)-1H-pyrrole-3-carboxamide peut être préparé comme décrit dans l'exemple 2 à partir de 2-amino-4-(4-nitro-phényl)-1H-pyrrole-3-carbonitrile.

### Exemple 13

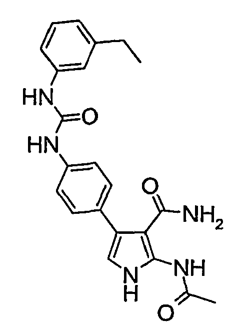

2-acétylamino-4-{4-[3-(3-éthyl-phényl)-uréido]-phényl}-1H-pyrrole-3-carboxamide
   0,061 cm³ (0,426 mmol) de 1-éthyl-3-isocyanato-benzène est ajouté à température ambiante à une suspension de 100 mg (0,387 mmol) de 2-acétylamino-4-(4-amino-phényl)-1H-pyrrole-3-carboxamide dans 5 cm³ de tétrahydrofurane anhydre. Le milieu réactionnel est agité à température ambiante pendant 24 heures puis évaporé à sec sous pression réduite. Le produit brut est purifié par chromatographie-flash [éluant : dichlorométhane /méthanol (97/3 en volume)]. Après concentration sous pression réduite des fractions contenant le produit attendu, on obtient 107 mg de 2-acétylamino-4-{4-[3-(3-éthyl-phényl)-uréido]-phényl}-1H-pyrrole-3-carboxamide sous forme d'un solide marron.
   LCMS (méthode A) : m/z = 406 : [M+H]⁺
   m/z = 389 : [M+H]+ - NH₃
   Temps de rétention (min.) = 3,67

### Exemple 14

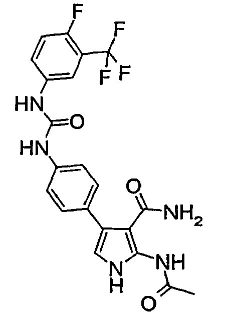

2-acétylamino-4-{4-[3-(4-fluoro-3-trifluorométhyl-phényl)-uréido]-phényl}-1H-pyrrole-3-carboxamide
   Le 2-acétylamino-4-{4-[3-(4-fluoro-3-trifluorométhyl-phényl)-uréido]-phényl}-1 H-pyrrole-3-carboxamide peut être préparé comme décrit dans l'exemple 13 à partir de 4-fluoro-3-(trifluorométhyl)phényl isocyanate et de 2-acétylamino-4-(4-amino-phényl)-1 H-pyrrole-3-carboxamide. Ses caractéristiques sont les suivantes :
   LCMS (méthode A) : m/z = 464 : [M+H]⁺; m/z 447 : [M+H]⁺ - NH₃ ( pic de base)
   m/z = 462 : [M-H]⁻
   Temps de rétention (min.) = 3,83

### Exemple 15

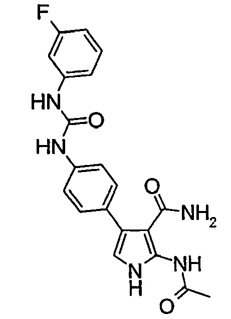

2-acétylamino-4-{4-[3-(3-fluoro-phényl)-uréido]-phényl}-1H-pyrrole-3-carboxamide
   Le 2-acétylamino-4-{4-[3-(3-fluoro-phényl)-uréido]-phényl}-1H-pyrrole-3-carboxamide peut être préparé comme décrit dans l'exemple 12 à partir de 3-fluorophényl isocyanate et du 2-acétylamino-4-(4-amino-phényl)-1H-pyrrole-3-carboxamide. Ses caractéristiques sont les suivantes :
   LCMS (méthode A) : m/z = 396 :[M+H]⁺ ; m/z = 379 : [M+H]⁺- NH₃
   Temps de rétention (min.) = 3,42

### Exemple 16

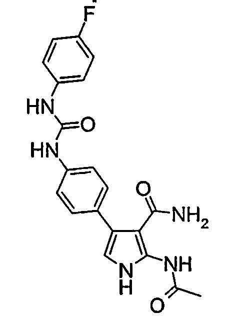

2-acétylamino-4-{4-[3-(4-fluoro-phényl)-uréido]-phényl}-1H-pyrrole-3-carboxamide
   Le 2-acétylamino-4-{4-[3-(4-fluoro-phényl)-uréido]-phényl}-1H-pyrrole-3-carboxamide peut être préparé comme décrit dans l'exemple 12 à partir de 4-fluorophényl isocyanate et de 2-acétylamino-4-(4-amino-phényl)-1H-pyrrole-3-carboxamide.
   LCMS (méthode A) : m/z = 396 : [M+H]⁺ ; m/z = 379 : [M+H]⁺ - NH₃
   Temps de rétention (min.) = 3,42

### Exemple 17

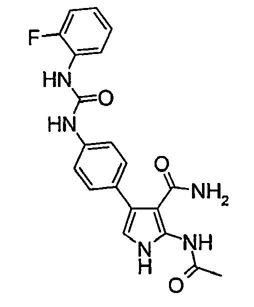

2-acétylamino-4-{4-[3-(2-fluoro-phényl)-uréido]-phényl}-1H-pyrrole-3-carboxamide
   Le 2-acétylamino-4-{4-[3-(2-fluoro-phényl)-uréido]-phényl}-1H-pyrrole-3-carboxamide peut être préparé comme décrit dans l'exemple 12 à partir de 2-fluorophényl isocyanate et de 2-acétylamino-4-(4-amino-phényl)-1H-pyrrole-3-carboxamide.
   LCMS (méthode B) : m/z = 396 : [M+H]⁺ (pic de base) ; m/z = 379 : [M+H]⁺ - NH₃
   Temps de rétention (min.) = 3,70

### Exemple 18

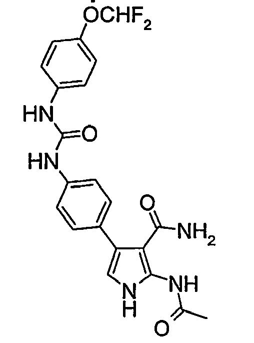

2-acétylamino-4-{4-[3-(4-difluorométhoxy-phényl)-uréido]-phényl}-1H-pyrrole-3-carboxamide
   Le 2-acétylamino-4-{4-[3-(4-difluorométhoxy-phényl)-uréido]-phényl}-1H-pyrrole-3-carboxamide peut être préparé comme décrit dans l'exemple 12 à partir de 4-(difluorométhoxy)phényl isocyanate et de 2-acétylamino-4-(4-amino-phényl)-1H-pyrrole-3-carboxamide.
   LCMS (méthode A) : m/z =444 :[M+H]⁺; m/z 427 : [M+H]⁺ - NH₃
   m/z = 442 : [M-H]⁻
   Temps de rétention (min.) = 3,51

### Exemple 19

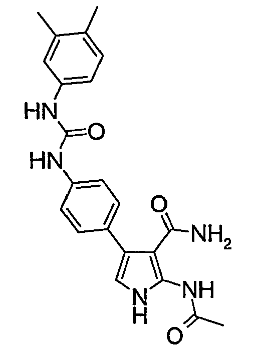

2-acétylamino-4-{4-[3-(3,4-diméthyl-phényl)-uréido]-phényl}-1H-pyrrole-3-carboxamide
   Le 2-acétylamino-4-{4-[3-(3,4-diméthyl-phényl)-uréido]-phényl}-1H-pyrrole-3-carboxamide peut être préparé comme décrit dans l'exemple 12 à partir de 3,4-diméthylphényl isocyanate et de 2-acétylamino-4-(4-amino-phény)-1H-pyrrole-3-carboxamide.
   LCMS (méthode A) : m/z = 406 : [M+H]⁺ , m/z = 389 : [M+H]⁺ - NH₃ m/z = 404 : [M-H]⁻
   Temps de rétention (min.) = 3,60

### Exemple 20

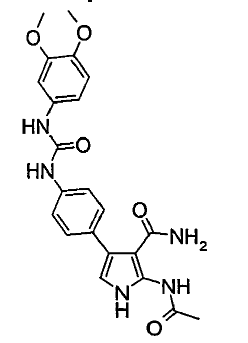

2-acétylamino-4-{4-[3-(3,4-diméthoxy-phényl)-uréido]-phényl}-1H-pyrrole-3-carboxamide
   Le 2-acétylamino-4-{4-[3-(3,4-diméthoxy-phényl)-uréido]-phényl}-1H-pyrrole-3-carboxamide peut être préparé comme décrit dans l'exemple 12 à partir de 3,4-diméthoxyphényl isocyanate et de 2-acétylamino-4-(4-amino-phényl)-1H-pyrrole-3-carboxamide.
   LCMS (méthode A) : m/z = 438 : [M+H]⁺ ; m/z = 421 : [M+H]⁺ - NH₃
   m/z = 436 : [M-H]⁻
   Temps de rétention (min.) = 2,94

### Exemple 21

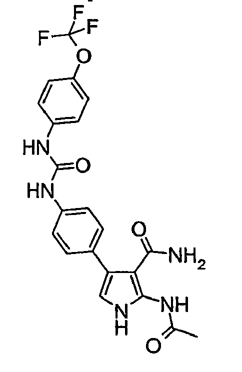

2-acétylamino-4-{4-[3-(4-trifluorométhoxy-phényl)-uréido]-phényl}-1 H-pyrrole-3-carboxamide
   Le 2-acétylamino-4-{4-[3-(4-trifluorométhoxy-phényl)-uréido]-phényl}-1 H-pyrrole-3-carboxamide peut être préparé comme décrit dans l'exemple 12 à partir de 4-(trifluorométhoxy)phényl isocyanate et de 2-acétylamino-4-(4-amino-phényl)-1H-pyrrole-3-carboxamide.
   LCMS (méthode A) : m/z = 462 : [M+H]⁺ ; m/z = 445 : [M+H]⁺ - NH₃
   m/z = 460 : [M-H]⁻
   Temps de rétention (min.) = 3,85

### Exemple 22

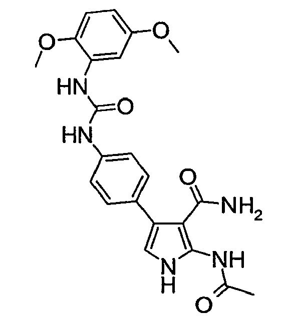

2-acétylamino-4-{4-[3-(2,5-diméthoxy-phényl)-uréido]-phényl}-1 H-pyrrole-3-carboxamide
   Le 2-acétylamino-4-{4-[3-(2,5-diméthoxy-phényl)-uréido]-phényl}-1H-pyrrole-3-carboxamide peut être préparé comme décrit dans l'exemple 13 à partir de 2,5-diméthoxyphényl isocyanate et de 2-acétylamino-4-(4-amino-phényl)-1 H-pyrrole-3-carboxamide.
   LCMS (méthode C) : m/z = 438 : [M+H]⁺
   m/z = 436 : [M-H]⁻
   Temps de rétention (min.) = 3,16

### Exemple 23

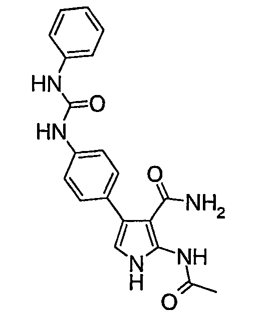

2-acétylamino-4-[4-(3-phényl-uréido)-phényl]-1H-pyrrol e-3-carboxamide
   Le 2-acétylamino-4-[4-(3-phényl-uréido)-phényl]-1H-pyrrole-3-carboxamide peut être préparé comme décrit dans l'exemple 13 à partir de phényl isocyanate et de 2-acétylamino-4-(4-amino-phényl)-1H-pyrrole-3-carboxamide.
   LCMS (méthode C) : m/z = 378 : [M+H]⁺
   m/z = 376 : [M-H]⁻
   Temps de rétention (min.) = 2,97

### Exemple 24

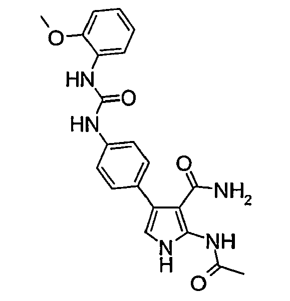

2-acétylamino-4-{4-[3-(2-méthoxy-phényl)-uréido]-phényl}-1H-pyrrole-3-carboxamide
   Le 2-acétylamino-4-{4-[3-(2-méthoxy-phényl)-uréido]-phényl}-1H-pyrrole-3-carboxamide peut être préparé comme décrit dans l'exemple 13 à partir de 2-méthoxyphényl isocyanate et de 2-acétylamino-4-(4-amino-phényl)-1H-pyrrole-3-carboxamide.
   LCMS (méthode C) : m/z = 408 : [M+H]⁺
   m/z = 406 : [M-H]⁻
   Temps de rétention (min.) = 3,16

### Exemple 25

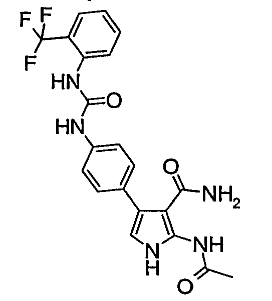

2-Acetylamino-4-{4-[3-(2-trifluorométhyl-phényl)-uréido]-phényl}-1H-pyrrole-3-carboxamide
   Le 2-acétylamino-4-{4-[3-(2-trifluorométhyl-phényl)-uréido]-phényl}-1H-pyrrole-3-carboxamide peut être préparé comme décrit dans l'exemple 13 à partir de 2-(trifluorométhyl)phényl isocyanate et de 2-acétylamino-4-(4-aminophényl)-1H-pyrrole-3-carboxamide.
   LCMS (méthode C) : m/z = 446 : [M+H]⁺
   m/z = 444 : [M-H]⁻
   Temps de rétention (min.) = 3,32

### Exemple 26

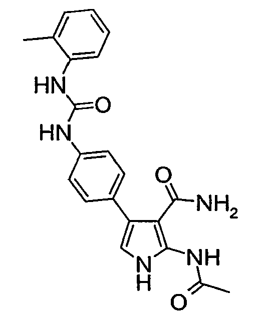

2-acétylamino-4-[4-(3-o-tolyl-uréido)-phényl]-1H-pyrrole-3-carboxamide
   Le 2-acétylamino-4-[4-(3-o-tolyl-uréido)-phényl]-1H-pyrrole-3-carboxamide peut être préparé comme décrit dans l'exemple 13 à partir de 2-méthylphényl isocyanate et de 2-acétylamino-4-(4-amino-phényl)-1H-pyrrole-3-carboxamide.
   LCMS (méthode C) : m/z = 392 : [M+H]⁺
   m/z = 390 : [M-H]⁻
   Temps de rétention (min.) = 3,07

### Exemple 27

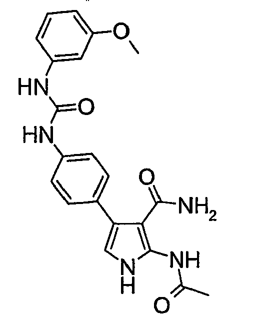

2-acétylamino-4-{4-[3-(3-méthoxy-phényl)-uréido]-phényl}-1H-pyrrole-3-carboxamide
   Le 2-acétylamino-4-{4-[3-(3-méthoxy-phényl)-uréido]-phényl}-1H-pyrrole-3-carboxamide peut être préparé comme décrit dans l'exemple 13 à partir de 3-méthoxyphényl isocyanate et de 2-acétylamino-4-(4-amino-phényl)-1H-pyrrole-3-carboxamide.
   LCMS (méthode C) : m/z = 408 : [M+H]⁺
   m/z = 406 : [M-H]⁻
   Temps de rétention (min.) = 3,01

### Exemple 28

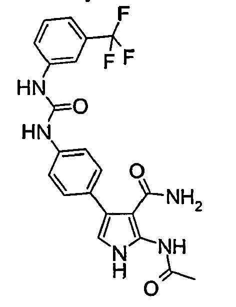

2-acétylamino-4-{4-[3-(3-trifluorométhyl-phényl)-uréido]-phényl}-1H-pyrrole-3-carboxamide
   Le 2-acétylamino-4-{4-[3-(3-trifluorométhyl-phényl)-uréido]-phényl}-1H-pyrrole-3-carboxamide peut être préparé comme décrit dans l'exemple 13 à partir de 3-(trifluorométhyl)phényl isocyanate et de 2-acétylamino-4-(4-aminophényl)-1 H-pyrrole-3-carboxamide.
   LCMS (méthode C) : m/z = 446 : [M+H]⁺
   m/z = 444 : [M-H]⁻
   Temps de rétention (min.) = 3,54

### Exemple 29

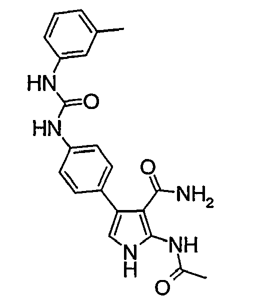

2-acétylamino-4-[4-(3-m-tolyl-uréido)-phényl]-1H-pyrrole-3-carboxamide
   Le 2-acétylamino-4-[4-(3-m-tolyl-uréido)-phényl]-1H-pyrrole-3-carboxamide peut être préparé comme décrit dans l'exemple 13 à partir de 3-méthylphényl isocyanate et de 2-acétylamino-4-(4-amino-phényl)-1H-pyrrole-3-carboxamide.
   LCMS (méthode C) : m/z = 392 : [M+H]⁺
   m/z = 390 : [M-H]⁻
   Temps de rétention (min.) = 3,20

### Exemple 30

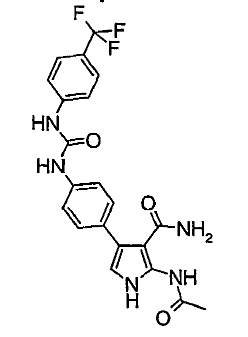

2-acétylamino-4-{4-[3-(4-trifluorométhyl-phényl)-uréido]-phényl}-1H-pyrrole-3-carboxamide
   Le 2-acétylamino-4-{4-[3-(4-trifluorométhyl-phényl)-uréido]-phényl}-1H-pyrrole-3-carboxamide peut être préparé comme décrit dans l'exemple 13 à partir de 4-(trifluorométhyl)phényl isocyanate et de 2-acétylamino-4-(4-aminophényl)-1H-pyrrole-3-carboxamide.
   LCMS (méthode C) : m/z = 446 : [M+H]⁺
   m/z = 444 : [M-H]⁻
   Temps de rétention (min.) = 3,58

### Exemple 31

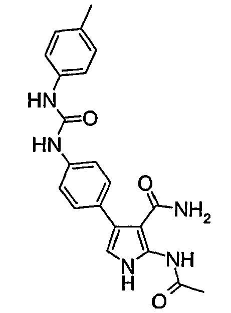

2-acétylamino-4-[4-(3-p-tolyl-uréido)-phényl]-1H-pyrrole-3-carboxamide
   Le 2-acétylamino-4-[4-(3-p-tolyl-uréido)-phényl]-1H-pyrrole-3-carboxamide peut être préparé comme décrit dans l'exemple 13 à partir de 4-méthylphényl isocyanate et de 2-acétylamino-4-(4-amino-phényl)-1H-pyrrole-3-carboxamide.
   LCMS (méthode C) : m/z = 392 : [M+H]⁺
   m/z =390 : [M-H]⁻
   Temps de rétention (min.) = 3,19

### Exemple 32

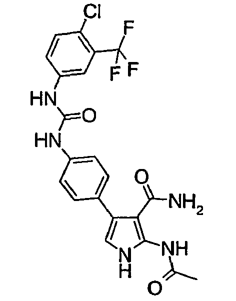

2-acétylamino-4-{4-[3-(4-chloro-3-trifluorométhyl-phényl)-uréido]-phényl}-1H-pyrrole-3-carboxamide
   Le 2-acétylamino-4-{4-[3-(4-chloro-3-trifluorométhyl-phényl)-uréido]-phényl}-1H-pyrrole-3-carboxamide peut être préparé comme décrit dans l'exemple 13 à partir de 4-chloro-3-(trifluorométhyl)phényl isocyanate et de 2-acétylamino-4-(4-amino-phényl)-1H-pyrrole-3-carboxamide.
   LCMS (méthode C) : m/z = 480 : [M+H]⁺
   m/z 478 = [M-H]⁻
   Temps de rétention (min.) = 3,80

### Exemple 33

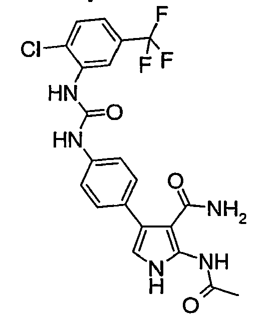

2-acétylamino-4-{4-[3-(2-chloro-5-trifluorométhyl-phényl)-uréido]-phényl}-1H-pyrrole-3-carboxamide
   Le 2-acétylamino-4-{4-[3-(2-chloro-5-trifluorométhyl-phényl)-uréido]-phényl}-1H-pyrrole-3-carboxamide peut être préparé comme décrit dans l'exemple 13 à partir de 2-chloro-5-(trifluorométhyl)phényl isocyanate et de 2-acétylamino-4-(4-amino-phényl)-1H-pyrrole-3-carboxamide.
   LCMS (méthode C) : m/z = 480 : [M+H]⁺
   m/z = 478 : [M-H]⁻
   Temps de rétention (min.) = 3,82

### Exemple 34

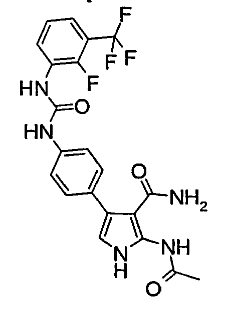

2-acétylamino-4-{4-[3-(2-fluoro-3-trifluorométhyl-phényl)-uréido]-phényl}-1H-pyrrole-3-carboxamide
   Le 2-acétylamino-4-{4-[3-(2-fluoro-3-trifluorométhyl-phényl)-uréido]-phényl}-1H-pyrrole-3-carboxamide peut être préparé comme décrit dans l'exemple 13 à partir de 2-fluoro-3-(trifluorométhyl)phényl isocyanate et de 2-acétylamino-4-(4-amino-phényl)-1H-pyrrole-3-carboxamide.
   LCMS (méthode C) : m/z = 464 : [M+H]⁺
   m/z = 462 : [M-H]⁻
   Temps de rétention (min.) = 3,64

### Exemple 35

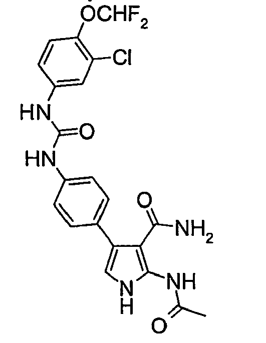

2-acétylamino-4-{4-[3-(3-chloro-4-difluorométhoxy-phényl)-uréido]-phényl}-1H-pyrrole-3-carboxamide
   Le 2-acétylamino-4-{4-[3-(3-chloro-4-difluorométhoxy-phényl)-uréido]-phényl}-1H-pyrrole-3-carboxamide peut être préparé comme décrit dans l'exemple 13 à partir de 3-chloro-4-(difluorométhoxy)phényl isocyanate et de 2-acétylamino-4-(4-amino-phényl)-1H-pyrrole-3-carboxamide.
   LCMS (méthode C) : m/z = 478 : [M+H]⁺
   m/z = 476 : [M-H]⁻
   Temps de rétention (min.) = 3,52

### Exemple 36

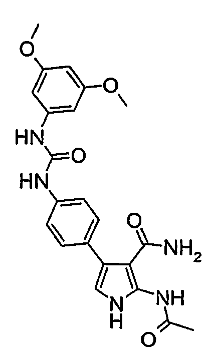

2-acétylamino-4-{4-[3-(3,5-diméthoxy-phényl)-uréido]-phényl}-1H-pyrrole-3-carboxamide
   Le 2-acétylamino-4-{4-[3-(3,5-diméthoxy-phényl)-uréido]-phényl}-1H-pyrrole-3-carboxamide peut être préparé comme décrit dans l'exemple 13 à partir de 3,5-diméthoxyphényl isocyanate et de 2-acétylamino-4-(4-amino-phényl)-1H-pyrrole-3-carboxamide.
   LCMS (méthode C) : m/z = 438 : [M+H]⁺
   m/z = 436 : [M-H]⁻
   Temps de rétention (min.) = 3,07

### Exemple 37

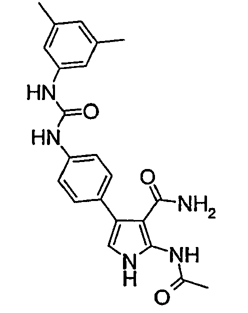

2-acétylamino-4-{4-[3-(3,5-diméthyl-phényl)-uréido]-phényl}-1H-pyrrole-3-carboxamide
   Le 2-acétylamino-4-{4-[3-(3,5-diméthyl-phényl)-uréido]-phényl}-1H-pyrrole-3-carboxamide peut être préparé comme décrit dans l'exemple 13 à partir de 3,5-diméthylphényl isocyanate et de 2-acétylamino-4-(4-amino-phényl)-1H-pyrrole-3-carboxamide.
   LCMS (méthode C) : m/z = 406 : [M+H]⁺
   m/z = 404 : [M-H]⁻
   Temps de rétention (min.) = 3,43

### Exemple 38

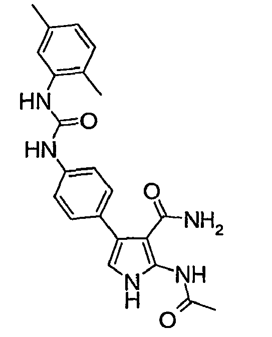

2-acétylamino-4-{4-[3-(2,5-diméthyl-phényl)-uréido]-phényl}-1H-pyrrole-3-carboxamide
   Le 2-acétylamino-4-{4-[3-(2,5-diméthyl-phényl)-uréido]-phényl}-1H-pyrrole-3-carboxamide peut être préparé comme décrit dans l'exemple 13 à partir de 2,5-diméthylphényl isocyanate et de 2-acétylamino-4-(4-amino-phényl)-1H-pyrrole-3-carboxamide.
   LCMS (méthode C) : m/z = 406 : [M+H]⁺
   m/z = 404 : [M-H]⁻
   Temps de rétention (min.) = 3,29

### Exemple 39

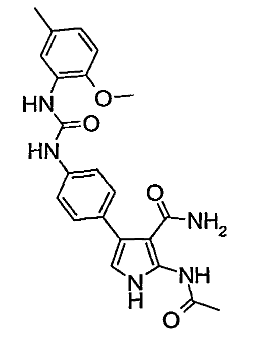

2-acétylamino-4-{4-[3-(2-méthoxy-5-méthyl-phényl)-uréido]-phényl}-1H-pyrrole-3-carboxamide
   Le acétylamino-4-{4-[3-(2-méthoxy-5-méthyl-phényl)-uréido]-phényl}-1H-pyrrole-3-carboxamide peut être préparé comme décrit dans l'exemple 13 à partir de 2-méthoxy-5-méthylphényl isocyanate et de 2-acétylamino-4-(4-amino-phényl)-1H-pyrrole-3-carboxamide.
   LCMS (méthode C) : m/z = 422 : [M+H]⁺
   m/z = 420 : [M-H]⁻
   Temps de rétention (min.) = 3,38

### Exemple 40

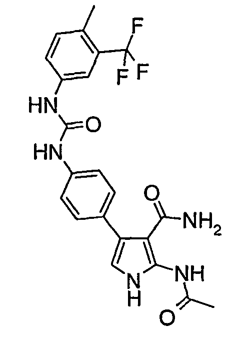

2-acétylamino-4-{4-[3-(4-méthyl-3-trifluorométhyl-phényl)-uréido]-phényl}-1H-pyrrole-3-carboxamide
   Le 2-acétylamino-4-{4-[3-(4-méthyl-3-trifluorométhyl-phényl)-uréido]-phényl}-1 H-pyrrole-3-carboxamide peut être préparé comme décrit dans l'exemple 13 à partir de 3-(trifluorométhyl)-4-méthylphényl isocyanate et de 2-acétylamino-4-(4-amino-phényl)-1H-pyrrole-3-carboxamide.
   Point de fusion = 266°C.
   ES⁺ : m/z = 460 : [M+H]⁺ ; m/z = 482 : [M+Na]⁺

### Exemple 41

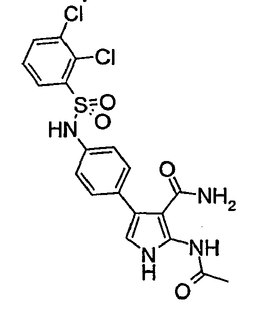

2-acétylamino-4-[4-(2,3-dichloro-benzènesulfonylamino)-phényl]-1H-pyrrole-3-carboxamide
   Une suspension de 70 mg (0.271 mmol) de 2-acétylamino-4-(4-aminophényl)-1H-pyrrole-3-carboxamide et de 70 mg (0.285 mmol) de 2,3-dichlorobenzènesulfonyl chloride dans 1.5 cm³ de pyridine est agitée à température ambiante pendant 24 heures puis le milieu réactionnel est évaporé à sec sous pression réduite. Le produit brut est purifié par chromatographie-flash [éluant: dichlorométhane / méthanol (97/3 en volume)]. Après concentration sous pression réduite des fractions contenant le produit attendu, on obtient 22 mg de 2-Acetylamino-4-[4-(2,3-dichloro-benzenesulfonylamino)-phényl]-1H-pyrrole-3-carboxamide sous forme d'un solide beige fondant à 265°C.
   ES⁺ : m/z = 469 : [M+H]⁺

### Détermination de l'activité des composés - Protocoles expérimentaux

### 1. FAK

L'activité inhibitrice des composés sur FAK est déterminée par une mesure de l'inhibition de l'autophosphorylation de l'enzyme en utilisant un test de fluorescence résolue dans le temps (HTRF).

L'ADNc complet de FAK humain, dont l'extrémité N-terminale a été marquée à l'histidine, a été cloné dans un vecteur d'expression baculovirus pFastBac HTc. La protéine a été exprimée et purifiée à environ 70% d'homogénéité.

L'activité kinase est déterminée en incubant l'enzyme (6.6 µg/ml) avec différentes concentrations de composé à tester dans un tampon 50 mM Hepes pH = 7,2, 10 mM MgCl₂, 100 µM Na₃VO₄ ,15 µM d'ATP pendant 1 heure à 37°C. La réaction enzymatique est stoppée par l'addition de tampon Hepes pH = 7,0 contenant 0.4 mM KF, 133 mM EDTA, 0.1 % BSA et le marquage est effectuée, pendant 1 à 2 heures à température ambiante, par l'addition dans ce tampon d'un anticorps anti-Histidine marqué avec XL665 et d'un anticorps monoclonal phosphospécifique de la tyrosine conjugué à du cryptate d'europium (Eu-K). Les caractéristiques des deux fluorophores sont disponibles dans G. Mathis et al., Anticancer Research, 1997, 17, pages 3011-3014. Le transfert d'énergie entre le cryptate d'europium excité vers le XL665 accepteur est proportionnel au degré d'autophosphorylation de FAK.

Le signal de longue durée spécifique de XL-665 est mesuré dans un compteur de plaques Packard Discovery. Tous les essais sont effectués en double exemplaire et la moyenne des deux essais est calculée. L'inhibition de l'activité d'autophosphorylation de FAK avec des composés de l'invention est exprimée en pourcentage d'inhibition par rapport à un contrôle dont l'activité est mesurée en l'absence de composé test. Pour le calcul du % d'inhibition, le ratio [signal à 665 nm/signal à 620 nm] est considéré.

### 2. KDR

L'effet inhibiteur des composés est déterminé dans un test de phosphorylation de substrat par l'enzyme KDR *in vitro* par une technique de scintillation (plaque 96 puits, NEN).
Le domaine cytoplasmique de l'enzyme KDR humaine a été cloné sous forme de fusion GST dans le vecteur d'expression baculovirus pFastBac. La protéine a été exprimée dans les cellules SF21 et purifiée à environ 60 % d'homogénéité.

L'activité kinase de KDR est mesurée dans 20 mM MOPS, 10 mM MgCl2, 10 mM MnCl2, 1 mM DTT, 2.5 mM EGTA, 10 mM b-glycérophosphate, pH = 7.2, en présence de 10 mM MgCl₂, 100 µM Na₃VO₄, 1 mM NaF. 10 µl du composé sont ajoutés à 70 µl de tampon kinase contenant 100 ng d'enzyme KDR à 4°C. La réaction est lancée en ajoutant 20 µl de solution contenant 2 µg de substrat (fragment SH2-SH3 de la PLC□ exprimée sous forme de protéine de fusion GST), 2 µCi □ ³³P[ATP] et 2 µM ATP froid. Après 1 heure d'incubation à 37°C, la réaction est stoppée en ajoutant 1 volume (100 µl) de 200 mM EDTA. Le tampon d'incubation est retiré, et les puits sont lavés trois fois avec 300 µl de PBS. La radioactivité est mesurée dans chaque puits en utilisant un compteur de radioactivité Top Count NXT (Packard).
Le bruit de fond est déterminé par la mesure de la radioactivité dans quatre puits différents contenant l'ATP radioactif et le substrat seul.
Un contrôle d'activité totale est mesuré dans quatre puits différents contenant tous les réactifs (γ³³P-[ATP], KDR et substrat PLCγ) mais en l'absence de composé.
L'inhibition de l'activité KDR avec le composé de l'invention est exprimée en pourcentage d'inhibition de l'activité contrôle déterminée en l'absence de composé.

Le composé SU5614 (Calbiochem) (1 µM) est inclus dans chaque plaque comme contrôle d'inhibition.

### 3. Tie2

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.
L'effet inhibiteur des molécules est déterminé dans un test de phosphorylation de PLC par Tie2 en présence de GST-Tie2 purifiée à environ 80% d'homogénéité. Le substrat est composé des fragments SH2-SH3 de la PLC exprimée sous forme de protéine de fusion GST.
L'activité kinase de Tie2 est mesurée dans un tampon MOPS 20mM pH 7.2, contenant 10 mM MgCl₂, 10 mM MnCl₂, 1 mM DTT, 10 mM de glycérophosphate. Dans une plaque 96 puits FlashPlate maintenue sur glace, on dépose un mélange réactionnel composé de 70 µl de tampon kinase contenant 100 ng d'enzyme GST-Tie2 par puits. Ensuite 10 µl de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi d'³³P[ATP]. Après 1 heure d'incubation à 37°C, la réaction est stoppée en ajoutant 1 volume (100µl) d'EDTA à 200 mM. Après élimination du tampon d'incubation, les puits sont lavés trois fois avec 300 µl de PBS. La radioactivité est mesurée sur un MicroBeta1450 Wallac.
L'inhibition de l'activité Tie2 est calculée et exprimée en pourcentage d'inhibition par rapport à l'activité contrôle déterminée en l'absence de composé.

**Résultats : Tableau 1 :**

| Structure | exemple | FAK | KDR | TIE2 |
|---|---|---|---|---|
| | | IC 50 (nM) | IC 50 (nM) | IC 50 (nM) |
| 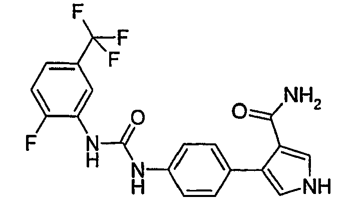 | **1** | 2055 | 6475 | 69 |
| 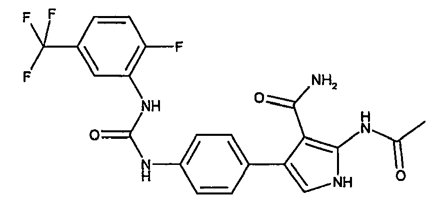 | **2** | 60 | 11.4 | 6.3 |
| 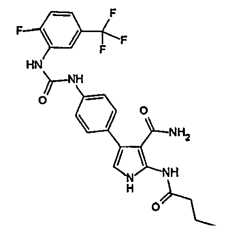 | **5** | 266 | 42 | 11 |
| 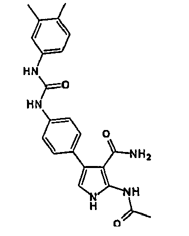 | **19** | 717 | 13 | 125 |
| 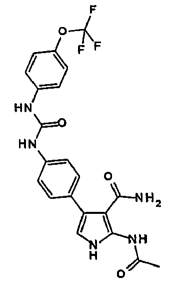 | **21** | 163 | 19 | 118 |

## Revendications

1. Produit répondant à la formule (I) suivante : 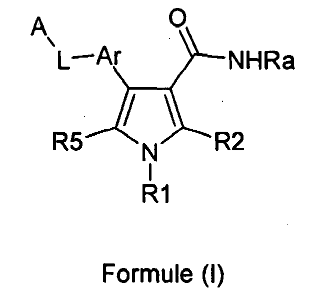 dans laquelle :
1) Ar-L-A est : 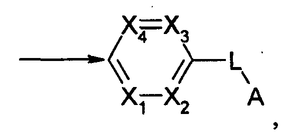 dans lequel chaque X1, X2, X3 et X4 est indépendamment choisi parmi N et CH, dans lequel A est choisi parmi phényle, pyrazolyle et isoxazolyle éventuellement substitué
2) L est NH-CO-NH,
3) Ra est H,
4) R1 est H,
5) R2 et R5 sont indépendamment sélectionnés dans le groupe constitué par: H, halogène, R'2, OR'2, NHR'2, NHCOR'2, NHCONHR'2, NHSO₂R'2 dans lequel chaque R'2, est sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué.

2. Produit selon la revendication 1, **caractérisé en ce que** R2 est H.

3. Produit selon la revendication 1, **caractérisé en ce que** R5 est H.

4. Produit selon la revendication 1, **caractérisé en ce que** A est substitué par un premier substituant sélectionné dans le groupe constitué par alkyle, alkyle halogéné, alkylène, alkynyle, aryle, hétéroaryle, O-alkyle, O-Aryle, O-hétéroaryle, S-alkyle, S-alkyle substitué, S-Aryle, S-hétéroaryle, chacun étant éventuellement substitué par un substituant choisi parmi (C1-C3)alkyle, halogène, O-(C1-C3)alkyle.

5. Produit selon l'une quelconque des revendications 1 à 4, **caractérisé en ce que caractérisé en ce que** A est substitué par un deuxième substituant sélectionné dans le groupe constitué par F, CI, Br, I, OH, SH, SO₃M, COOM, CN, NO₂, CON(R8)(R9), N(R8)CO(R9), (C1-C3)alkyle-OH, (C1-C3)alkyleN(R8)(R9), (C1-C3)alkyle-(R10), (C1-C3)alkyle-COOH, N(R8)(R9); dans lequel R8 et R9 sont indépendamment choisis parmi H, (C1-C3)alkyle, (C1-C3)alkyle halogéné, (C1-C3)alkyleOH, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons contenant de 0 à 3 hétéroatomes choisis parmi N, O et S; dans lequel M est H ou un cation de métal alcalin choisi parmi Li, Na et K ; et dans lequel R10 est H ou un hétérocycle non aromatique éventuellement substitué comprenant de 2 à 7 atomes de carbone, et de 1 à 3 hétéroatomes choisis parmi N, O et S.

6. Produit selon l'une quelconque des revendications 1 à 5 **caractérisé en ce qu'**il s'agit de :
4-{4-[3-(2-Fluoro-5-trifluorométhyl-phényl)-uréido]-phényl}-1H-pyrrole-3-carboxamide,
1-Acétyl-2-amino-4-{4-[3-(2-fluoro-5-trifluorométhyl-phényl)-ureido]-phényl}-1H-pyrrole-3-carboxamide,
2-Formylamino-4-{4-[3-(2-fluoro-5-trifluorométhyl-phényl)-uréido]-phényl}-1H-pyrrole-3-carboxamide,
2-Isobutyrylamino-4-{4-[3-(2-fluoro-5-trifluorométhyl-phényl)-uréido]-phényl}-1H-pyrrole-3-carboxamide,
2-Butyrylamino-4-{4-[3-(2-fluoro-5-trifluorométhyl-phényl)-uréido]-phényl}-1H-pyrrole-3-carboxamide,
2-(3-Cyclopentyl-propionylamino)-4-{4-[3-(2-fluoro-5-trifluorométhyl-phényl)-uréido]-phényl}-1H-pyrrole-3-carboxamide,
2-(Cyclopropylcarbonylamino)-4-{4-[3-(2-fluoro-5-trifluorométhyl-phényl)-uréido]-phényl}-1H-pyrrole-3-carboxamide,
2-Pivaloylamino-4-{4-[3-(2-fluoro-5-trifluorométhyl-phényl)-uréido]-phényl}-1H-pyrrole-3-carboxamide,
2-(2-Diméthylamino-acetylamino)--4-{4-[3-(2-fluoro-5-trifluorométhyl-phényl)-uréido]-phényl}-1H-pyrrole-3-carboxamide,
2-Acétylamino-4-{6-[3-(2-fluoro-5-trifluorométhyl-phényl)-uréido]-pyridin-3-yl}-1H-pyrrole-3-carboxamide,
2-(3-Éthyl-uréido)-4-{4-[3-(2-fluoro-5-trifluorométhyl-phényl)-uréido]-phényl}-1H-pyrrole-3-carboxamide,
2-acétylamino-4-{4-[3-(3-fluoro-5-trifluorométhyl-phényl)-uréido]-phényl}-1H-pyrrole-3-carboxamide,
2-acétylamino-4-{4-[3-(3-éthyl-phényl)-uréido]-phényl}-H-pyrrole-3-carboxamide,
2-acétylamino-4-{4-[3-(4-fluoro-3-trifluorométhyl-phényl)-uréido]-phényl}-1H-pyrrole-3-carboxamide,
2-acétylamino-4-{4-[3-(3-fluoro-phényl)-uréido]-phényl}-1H-pyrrole-3-carboxamide,
2-acétylamino-4-{4-[3-(4-fluoro-phényl)-uréido]-phényl}-1H-pyrrole-3-carboxamide,
2-acétylamino-4-{4-[3-(2-fluoro-phényl)-uréido]-phényl}-1H-pyrrole-3-carboxamide,
2-acétylamino-4-{4-[3-(4-difluorométhoxy-phényl)-uréido]-phényl}-1H-pyrrole-3-carboxamide,
2-acétylamino-4-{4-[3-(3,4-diméthyl-phényl)-uréido]-phényl}-1H-pyrrole-3-carboxamide,
2-acétylamino-4-{4-[3-(3,4-diméthoxy-phényl)-uréido]-phényl}-1H-pyrrole-3-carboxamide,
2-acétylamino-4-{4-[3-(4-trifluorométhoxy-phényl)-uréido]-phényl}-1H-pyrrole-3-carboxamide
2-acétylamino-4-{4-[3-(2,5-diméthoxy-phényl)-uréido]-phényl}-1H-pyrrole-3-carboxamide,
2-acétylamino-4-[4-(3-phényl-uréido)-phényl]-1H-pyrrole-3-carboxamide,
2-acétylamino-4-{4-[3-(2-méthoxy-phényl)-uréido]-phényl}-1H-pyrrole-3-carboxamide,
2-Acetylamino-4-{4-[3-(2-trifluorométhyl-phényl)-uréido]-phényl}-1H-pyrrole-3-carboxamide ,
2-acétylamino-4-[4-(3-o-tolyl-uréido)-phényl]-1H-pyrrole-3-carboxamide,
2-acétylamino-4-{4-[3-(3-méthoxy-phényl)-uréido]-phényl}-1H-pyrrole-3-carboxamide,
2-acétylamino-4-{4-[3-(3-trifluorométhyl-phényl)-uréido]-phényl}-1H-pyrrole-3-carboxamide,
2-acétylamino-4-[4-(3-m-tolyl-uréido)-phényl]-1H-pyrrole-3-carboxamide
2-acétylamino-4-{4-[3-(4-trifluorométhyl-phényl)-uréido]-phényl}-1H-pyrrole-3-carboxamide,
2-acétylamino-4-[4-(3-p-tolyl-uréido)-phényl]-1H-pyrrole-3-carboxa m ide,
2-acétylamino-4-{4-[3-(4-chloro-3-trifluorométhyl-phényl)-uréido]-phényl}-1H-pyrrole-3-carboxamide,
2-acétylamino-4-{4-[3-(2-chloro-5-trifluorométhyl-phényl)-uréido]-phényl}-1H-pyrrole-3-carboxamide,
2-acétylamino-4-{4-[3-(2-fluoro-3-trifluorométhyl-phényl)-uréido]-phényl}-1H-pyrrole-3-carboxamide,
2-acétylamino-4-{4-[3-(3-chloro-4-difluorométhoxy-phényl)-uréido]-phényl}-1H-pyrrole-3-carboxamide,
2-acétylamino-4-{4-[3-(3,5-diméthoxy-phényl)-uréido]-phényl}-1H-pyrrole-3-carboxamide,
2-acétylamino-4-{4-[3-(3,5-diméthyl-phényl)-uréido]-phényl}-1H-pyrrole-3-carboxamide,
2-acétylamino-4-{4-[3-(2,5-diméthyl-phényl)-uréido]-phényl}-1H-pyrrole-3-carboxamide,
2-acétylamino-4-{4-[3-(2-méthoxy-5-méthyl-phényl)-uréido]-phényl}-1H-pyrrole-3-carboxamide,
2-acétylamino-4-{4-[3-(4-méthyl-3-trifluorométhyl-phényl)-uréido]-phényl}-1H-pyrrole-3-carboxamide,
2-acétylamino-4-[4-(2,3-dichloro-benzénesulfonylamino)-phényl]-1H-pyrrole-3-carboxamide.

7. Produit selon l'une quelconque des revendications précédentes, **caractérisé en ce qu'**il est sous forme :
1) non chirale, ou
2) racémique, ou
3) enrichie en un stéréo-isomère, ou
4) enrichie en un énantiomère ;
et **en ce qu'**il est éventuellement salifié.

8. Composition pharmaceutique comprenant un produit selon l'une quelconque des revendications précédentes, en combinaison avec un excipient pharmaceutiquement acceptable.

9. Utilisation d'un produit selon l'une quelconque des revendications 1 à 8, pour la fabrication d'un médicament utile pour traiter le cancer.

## Claims

1. Product corresponding to the following formula (I): 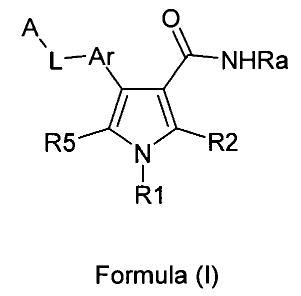 in which:
1) Ar-L-A is: 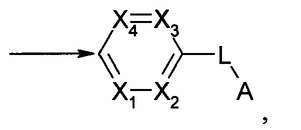 in which each X1, X2, X3 and X4 is independently chosen from N and CH,
in which A is chosen from optionally substituted phenyl, pyrazolyl and isoxazolyl;
2) L is NH-CO-NH;
3) Ra is H;
4) R1 is H;
5) R2 and R5 are independently selected from the group consisting of: H, halogen, R'2, OR'2, NHR'2, NHCOR'2, NHCONHR'2, NHSO₂R'2 in which each R'2, R'3, R'4 is independently selected from the group consisting of H, alkyl, alkylene, alkynyl, aryl, heteroaryl, cycloalkyl, heterocyclyl, substituted alkyl, substituted alkylene, substituted alkynyl, substituted aryl, substituted heteroaryl, substituted cycloalkyl, substituted heterocyclyl.

2. Product according to Claim 1, **characterized in that** R2 is H.

3. Product according to Claim 1, **characterized in that** R5 is H.

4. Product according to Claim 1, **characterized in that** A is substituted with a first substituent selected from the group consisting of alkyl, halogenated alkyl, alkylene, alkynyl, aryl, heteroaryl, O-alkyl, O-aryl, O-heteroaryl, S-alkyl, substituted S-alkyl, S-aryl, S-heteroaryl, each being optionally substituted with a substituent chosen from (C1-C3)alkyl, halogen, O-(C1-C3)alkyl.

5. Product according to any one of Claims 1 to 4, **characterized in that** A is substituted with a second substituent selected from the group consisting of F, Cl, Br, I, OH, SH, SO₃M, COOM, CN, NO₂, CON(R8)(R9), N(R8)CO(R9), (C1-C3)alkyl-OH, (C1-C3)alkyl-N(R8)(R9), (C1-C3)alkyl-(R10), (C1-C3)alkyl-COOH, N(R8)(R9); in which R8 and R9 are independently chosen from H, (C1-C3)alkyl, halogenated (C1-C3)alkyl, (C1-C3)alkylOH, (C1-C3)alkylNH₂, (C1-C3)alkylCOOM, (C1-C3)alkylSO₃M; in which, when R8 and R9 are simultaneously different from H, they may be linked to form a 5- to 7-membered ring containing from 0 to 3 heteroatoms chosen from N, O and S; in which M is H or an alkali metal cation chosen from Li, Na and K; and in which R10 is H or an optionally substituted nonaromatic heterocycle comprising 2 to 7 carbon atoms, and 1 to 3 heteroatoms chosen from N, O and S.

6. Product according to any one of Claims 1 to 5, **characterized in that** it is:
4-{4-[3-(2-fluoro-5-trifluoromethylphenyl)ureido]phenyl}-1H-pyrrole-3-carboxamide,
1-acetyl-2-amino-4-{4-[3-(2-fluoro-5-trifluoromethylphenyl)ureido]phenyl}-1H-pyrrole-3-carboxamide,
2-formylamino-4-{4-[3-(2-fluoro-5-trifluoromethylphenyl)ureido]phenyl}-1H-pyrrole-3-carboxamide,
2-isobutyrylamino-4-{4-[3-(2-fluoro-5-trifluoromethylphenyl)ureido]phenyl}-1H-pyrrole-3-carboxamide,
2-butyrylamino-4-{4-[3-(2-fluoro-5-trifluoromethylphenyl)ureido]phenyl}-1H-pyrrole-3-carboxamide,
2-(3-cyclopentylpropionylamino)-4-{4-[3-(2-fluoro-5-trifluoromethylphenyl)-ureido]phenyl}-1H-pyrrole-3-carboxamide,
2-(cyclopropylcarbonylamino)-4-{4-[3-(2-fluoro-5-trifluoromethylphenyl)-ureido]phenyl}-1H-pyrrole-3-carboxamide,
2-pivaloylamino-4-{4-[3-(2-fluoro-5-trifluoromethylphenyl)ureido]phenyl}-1H-pyrrole-3-carboxamide,
2-(2-dimethylaminoacetylamino)-4-{4-[3-(2-fluoro-5-trifluoromethylphenyl)-ureido]phenyl}-1H-pyrrole-3-carboxamide,
2-acetylamino-4-{6-[3-(2-fluoro-5-trifluoromethylphenyl)ureido]pyridin-3-yl}-1H-pyrrole-3-carboxamide,
2-(3-ethylureido)-4-{4-[3-(2-fluoro-5-trifluoromethylphenyl)ureido]phenyl}-1H-pyrrole-3-carboxamide,
2-acetylamino-4-{4-[3-(3-fluoro-5-trifluoromethylphenyl)ureido]phenyl}-1H-pyrrole-3-carboxamide,
2-acetylamino-4-{4-[3-(3-ethylphenyl)ureido]phenyl}-1H-pyrrole-3-carboxamide,
2-acetylamino-4-{4-[3-(4-fluoro-3-trifluoromethylphenyl)ureido]phenyl}-1H-pyrrole-3-carboxamide,
2-acetylamino-4-{4-[3-(3-fluorophenyl)ureido]phenyl}-1H-pyrrole-3-carboxamide,
2-acetylamino-4-{4-[3-(4-fluorophenyl)ureido]phenyl}-1H-pyrrole-3-carboxamide,
2-acetylamino-4-{4-[3-(2-fluorophenyl)ureido]phenyl}-1H-pyrrole-3-carboxamide,
2-acetylamino-4-{4-[3-(4-difluoromethoxyphenyl)ureido]phenyl}-1H-pyrrole-3-carboxamide,
2-acetylamino-4-{4-[3-(3,4-dimethylphenyl)ureido]phenyl}-1H-pyrrole-3-carboxamide,
2-acetylamino-4-{4-[3-(3,4-dimethoxyphenyl)ureido]phenyl}-1H-pyrrole-3-carboxamide,
2-acetylamino-4-{4-[3-(4-trifluoromethoxyphenyl)ureido]phenyl}-1H-pyrrole-3-carboxamide
2-acetylamino-4-{4-[3-(2,5-dimethoxyphenyl)ureido]phenyl}-1H-pyrrole-3-carboxamide,
2-acetylamino-4-[4-(3-phenylureido)phenyl]-1H-pyrrole-3-carboxamide,
2-acetylamino-4-{4-[3-(2-methoxyphenyl)ureido]phenyl}-1H-pyrrole-3-carboxamide,
2-acetylamino-4-{4-[3-(2-trifluoromethylphenyl)ureido]phenyl}-1H-pyrrole-3-carboxamide,
2-acetylamino-4-[4-(3-o-tolylureido)phenyl]-1H-pyrrole-3-carboxamide,
2-acetylamino-4-{4-[3-(3-methoxyphenyl)ureido]phenyl}-1H-pyrrole-3-carboxamide,
2-acetylamino-4-{4-[3-(3-trifluoromethylphenyl)ureido]phenyl}-1H-pyrrole-3-carboxamide,
2-acetylamino-4-[4-(3-m-tolylureido)phenyl]-1H-pyrrole-3-carboxamide
2-acetylamino-4-{4-[3-(4-trifluoromethylphenyl)ureido]phenyl}-1H-pyrrole-3-carboxamide,
2-acetylamino-4-[4-(3-p-tolylureido)phenyl]-1 H-pyrrole-3-carboxamide,
2-acetylamino-4-{4-[3-(4-chloro-3-trifluoromethylphenyl)ureido]phenyl}-1H-pyrrole-3-carboxamide,
2-acetylamino-4-{4-[3-(2-chloro-5-trifluoromethylphenyl)ureido]phenyl}-1H-pyrrole-3-carboxamide,
2-acetylamino-4-{4-[3-(2-fluoro-3-trifluoromethylphenyl)ureido]phenyl}-1H-pyrrole-3-carboxamide,
2-acetylamino-4-{4-[3-(3-chloro-4-difluoromethoxyphenyl)ureido]phenyl}-1H-pyrrole-3-carboxamide,
2-acetylamino-4-{4-[3-(3,5-dimethoxyphenyl)ureido]phenyl}-1H-pyrrole-3-carboxamide,
2-acetylamino-4-{4-[3-(3,5-dimethylphenyl)ureido]phenyl}-1H-pyrrole-3-carboxamide,
2-acetylamino-4-{4-[3-(2,5-dimethylphenyl)ureido]phenyl}-1H-pyrrole-3-carboxamide,
2-acetylamino-4-{4-[3-(2-methoxy-5-methylphenyl)ureido]phenyl}-1H-pyrrole-3-carboxamide,
2-acetylamino-4-{4-[3-(4-methyl-3-trifluoromethylphenyl)ureido]phenyl}-1H-pyrrole-3-carboxamide,
2-acetylamino-4-[4-(2,3-dichlorobenzenesulphonylamino)phenyl]-1H-pyrrole-3-carboxamide.

7. Product according to any one of the preceding claims, **characterized in that** it is in a form which is:
1) non-chiral, or
2) racemic, or
3) enriched with one stereoisomer, or
4) enriched with one enantiomer;
and **in that** it is optionally salified.

8. Pharmaceutical composition comprising a product according to any one of the preceding claims, in combination with a pharmaceutically acceptable excipient.

9. Use of a product according to any one of Claims 1 to 8, for the manufacture of a medicament useful for treating cancer.

## Patentansprüche

1. Produkt der folgenden Formel (I): 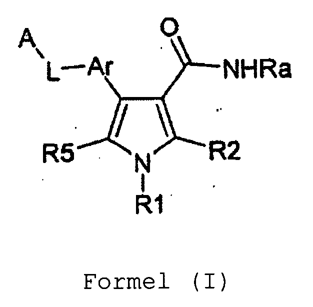 worin:
1) Ar-L-A für: 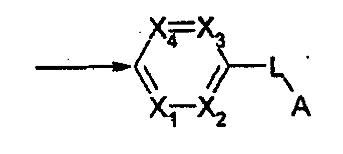 steht, worin X1, X2, X3 und X4 jeweils unabhängig voneinander unter N und CH ausgewählt sind,
worin A unter Phenyl, Pyrazolyl und Isoxazolyl, das gegebenenfalls substituiert sein kann, ausgewählt ist,
2) L für NH-CO-NH steht,
3) Ra für H steht,
4) R1 für H steht,
5) R2 und R5 unabhängig voneinander aus der Gruppe bestehend aus H, Halogen, R'2, OR'2, NHR'2, NHCOR'2, NHCONHR'2 und NHSO₂R'2 ausgewählt sind,
worin R'2 jeweils aus der Gruppe bestehend aus H, Alkyl, Alkylen, Alkinyl, Aryl, Heteroaryl, Cycloalkyl, Heterocyclyl, substituiertem Alkyl, substituiertem Alkylen, substituiertem Alkinyl, substituiertem Aryl, substituiertem Heteroaryl, substituiertem Cycloalkyl und substituiertem Heterocyclyl ausgewählt ist.

2. Produkt nach Anspruch 1, **dadurch gekennzeichnet, daß** R2 für H steht.

3. Produkt nach Anspruch 1, **dadurch gekennzeichnet, daß** R5 für H steht.

4. Produkt nach Anspruch 1, **dadurch gekennzeichnet, daß** A durch einen ersten Substituenten aus der Gruppe bestehend aus Alkyl, Halogenalkyl, Alkylen, Alkinyl, Aryl, Heteroaryl, O-Alkyl, O-Aryl, 0-Heteroaryl, S-Alkyl, substituiertem S-Alkyl, S-Aryl und S-Heteroaryl, jeweils gegebenenfalls substituiert durch einen unter (C₁-C₃)-Alkyl, Halogen und O-(C₁-C₃)-Alkyl ausgewählten Substituenten, substituiert ist.

5. Produkt nach einem der Ansprüche 1 bis 4, **dadurch gekennzeichnet, daß** A durch einen zweiten Substituenten aus der Gruppe bestehend aus F, Cl, Br, I, OH, SH, SO₃M, COOM, CN, NO₂, CON(R8)(R9), N(R8)CO(R9), (C1-C3)-Alkyl-OH, (C1-C3)-Alkyl-N(R8)(R9), (C₁-C₃)-Alkyl-(R10), (C₁-C₃)-Alkyl-COOH und N(R8)(R9) substituiert ist; worin R8 und R9 unabhängig voneinander unter H, (C₁-C₃)-Alkyl, Halogen- (C₁-C₃)-alkyl, (C₁-C₃)-Alkyl-OH, (C₁-C₃)-Alkyl-NH₂, (C₁-C₃)-Alkyl-COOM und (C₁-C₃)-Alkyl-SO₃M ausgewählt sind; worin R8 und R9 dann, wenn sie gleichzeitig von H verschieden sind, unter Bildung eines 5- bis 7-gliedrigen Rings mit 0 bis 3 unter N, 0 und S ausgewählten Heteroatomen miteinander verbunden sein können; worin M für H oder ein unter Li, Na und K ausgewähltes Alkalimetallkation steht und worin R10 für H oder einen gegebenenfalls substituierten nichtaromatischen Heterocyclus mit 2 bis 7 Kohlenstoffatomen und 1 bis 3 unter N, O und S ausgewählten Heteroatomen steht.

6. Produkt nach einem der Ansprüche 1 bis 5, **dadurch gekennzeichnet, daß** es sich dabei um:
4-{4-[3-(2-Fluor-5-trifluormethylphenyl)ureido]-phenyl}-1H-pyrrol-3-carboxamid,
1-Acetyl-2-amino-4-{4-[3-(2-fluor-5-trifluormethylphenyl)ureido]phenyl}-1H-pyrrol-3-carboxamid,
2-Formylamino-4-{4-[3-(2-fluor-5-trifluormethylphenyl)ureido]phenyl}-1H-pyrrol-3-carboxamid,
2-Isobutyrylamino-4-{4-[3-(2-fluor-5-trifluormethylphenyl)ureido]phenyl}-1H-pyrrol-3-carboxamid,
2-Butyrylamino-4-{4-[3-(2-fluor-5-trifluormethylphenyl)ureido]phenyl}-1H-pyrrol-3-carboxamid,
2-(3-Cyclopentylpropionylamino)-4-{4-[3-(2-fluor-5-trifluormethylphenyl)ureido]phenyl}-1H-pyrrol-3-carboxamid,
2-(Cyclopropylcarbonylamino)-4-{4-[3-(2-fluor-5-trifluormethylphenyl)ureido]phenyl}-1H-pyrrol-3-carboxamid,
2-Pivaloylamino-4-{4-[3-(2-fluor-5-trifluormethylphenyl)ureido]phenyl}-1H-pyrrol-3-carboxamid,
2-(2-Dimethylaminoacetylamino)-4-{4-[3-(2-fluor-5-trifluormethylphenyl)ureido]phenyl}-1H-pyrrol-3-carboxamid,
2-Acetylamino-4-{6-[3-(2-fluor-5-trifluormethylphenyl)ureido]pyridin-3-yl}-1H-pyrrol-3-carboxamid,
2-(3-Ethylureido)-4-{4-[3-(2-fluor-5-trifluormethylphenyl)ureido]phenyl}-1H-pyrrol-3-carboxamid,
2-Acetylamino-4-{4-[3-(3-fluor-5-trifluormethylphenyl)ureido]phenyl}-1H-pyrrol-3-carboxamid,
2-Acetylamino-4-{4-[3-(3-ethylphenyl)ureido]-phenyl}-1H-pyrrol-3-carboxamid,
2-Acetylamino-4-{4-[3-(4-fluor-3-trifluormethylphenyl)ureido]phenyl}-1H-pyrrol-3-carboxamid,
2-Acetylamino-4-{4-[3-(3-fluorphenyl)ureido]-phenyl}-1H-pyrrol-3-carboxamid,
2-Acetylamino-4-{4-[3-(4-fluorphenyl)ureido]-phenyl}-1H-pyrrol-3-carboxamid,
2-Acetylamino-4-{4-[3-(2-fluorphenyl)ureido]-phenyl}-1H-pyrrol-3-carboxamid,
2-Acetylamino-4-{4-[3-(4-difluormethoxyphenyl)-ureido]phenyl}-1H-pyrrol-3-carboxamid,
2-Acetylamino-4-{4-[3-(3,4-dimethylphenyl)ureido]-phenyl}-1H-pyrrol-3-carboxamid,
2-Acetylamino-4-{4-[3-(3,4-dimethoxyphenyl)-ureido]phenyl}-1H-pyrrol-3-carboxamid,
2-Acetylamino-4-{4-[3-(4-trifluormethoxyphenyl)-ureido]phenyl}-1H-pyrrol-3-carboxamid
2-Acetylamino-4-{4-[3-(2,5-dimethoxyphenyl)-ureido]phenyl}-1H-pyrrol-3-carboxamid,
2-Acetylamino-4-[4-(3-phenylureido)phenyl]-1H-pyrrol-3-carboxamid,
2-Acetylamino-4-{4-[3-(2-methoxyphenyl)ureido]-phenyl}-1H-pyrrol-3-carboxamid,
2-Acetylamino-4-{4-[3-(2-trifluormethylphenyl)-ureido]phenyl}-1H-pyrrol-3-carboxamid,
2-Acetylamino-4-[4-(3-o-tolylureido)phenyl]-1H-pyrrol-3-carboxamid,
2-Acetylamino-4-{4-[3-(3-methoxyphenyl)ureido]-phenyl}-1H-pyrrol-3-carboxamid,
2-Acetylamino-4-{4-[3-(3-trifluormethylphenyl)-ureido]phenyl}-1H-pyrrol-3-carboxamid,
2-Acetylamino-4-[4-(3-m-tolylureido)phenyl]-1H-pyrrol-3-carboxamid
2-Acetylamino-4-{4-[3-(4-trifluormethylphenyl)-ureido]phenyl}-1H-pyrrol-3-carboxamid,
2-Acetylamino-4-[4-(3-p-tolylureido)phenyl]-1H-pyrrol-3-carboxamid,
2-Acetylamino-4-{4-[3-(4-chlor-3-trifluormethylphenyl)ureido]phenyl}-1H-pyrrol-3-carboxamid,
2-Acetylamino-4-{4-[3-(2-chlor-5-trifluormethylphenyl)ureido]phenyl}-1H-pyrrol-3-carboxamid,
2-Acetylamino-4-{4-[3-(2-fluor-3-trifluormethylphenyl)ureido]phenyl}-1H-pyrrol-3-carboxamid,
2-Acetylamino-4-{4-[3-(3-chloro-4-difluormethoxy-phenyl)ureido]phenyl}-1H-pyrrol-3-carboxamid,
2-Acetylamino-4-{4-[3-(3,5-dimethoxyphenyl)-ureido]phenyl}-1H-pyrrol-3-carboxamid,
2-Acetylamino-4-{4-[3-(3,5-dimethylphenyl)ureido]-phenyl}-1H-pyrrol-3-carboxamid,
2-Acetylamino-4-{4-[3-(2,5-dimethylphenyl)ureido]-phenyl}-1H-pyrrol-3-carboxamid,
2-Acetylamino-4-{4-[3-(2-methoxy-5-methylphenyl)-ureido]phenyl}-1H-pyrrol-3-carboxamid,
2-Acetylamino-4-{4-[3-(4-methyl-3-trifluormethylphenyl)ureido]phenyl}-1H-pyrrol-3-carboxamid,
2-Acetylamino-4-[4-(2,3-dichlorbenzolsulfonyl-amino)phenyl]-1H-pyrrol-3-carboxamid handelt.

7. Produkt nach einem der vorhergehenden Ansprüche, **dadurch gekennzeichnet, daß** es:
1) in nichtchiraler Form,
2) in racemischer Form,
3) in mit einem Stereoisomer angereicherter Form oder
4) in mit einem Enantiomer angereicherter Form vorliegt und gegebenenfalls versalzt ist.

8. Pharmazeutische Zusammensetzung, enthaltend ein Produkt nach einem der vorhergehenden Ansprüche in Kombination mit einem pharmazeutisch annehmbaren Hilfsstoff.

9. Verwendung eines Produkts nach einem der Ansprüche 1 bis 8 zur Herstellung eines Arzneimittels, das zur Behandlung von Krebs verwendet werden kann.
